# EUROPEAN PATENT APPLICATION

(11) **EP 4 671 369 A2**
(43) Date of publication of application: **31.12.2025**
(21) Application number: 25187377.4
(22) Date of filing: 16.06.2017
(51) Int. Cl.: C12N 15/09

(54) **TREATMENT OF AMD USING AAV2 VARIANT WITH AFLIBERCEPT**

(30) Priority: 16.06.2016 US 201662351234 P
(62) Divisional of application: 20196657.9
(71) Applicant: Adverum Biotechnologies, Inc., Redwood City, CA 94063 (US)
(72) Inventor: BLUMENKRANZ, Mark, Redwood City, 94063 (US); GASMI, Mehdi, Redwood City, 94063 (US)
(74) Representative: EIP

(57) **Abstract**

The present disclosure provides pharmaceutical compositions and methods thereof for the treatment of age-related macular degeneration (AMD) in a subject by administering intravitreally a pharmaceutical composition adapted for gene therapy, comprising a vector having a nucleic acid sequence that encodes aflibercept.

## Description

### CROSS-REFERENCE

This application claims the benefit of U.S. Provisional Application No. 62/351,234, filed June 16, 2016, the disclosure of which is incorporated herein by reference in its entirety.

### BACKGROUND OF THE DISCLOSURE

Aflibercept is a recombinant fusion protein that acts as a decoy receptor for vascular endothelial growth factor subtypes A and B (VEGF-A and VEGF-B) and placental growth factor (PIGF). By binding to these ligands, aflibercept is able to prevent these ligands from binding to vascular endothelial growth factor receptors (VEGFR), VEGFR-1 and VEGFR-2, to suppress neovascularization and decrease vascular permeability. Aflibercept consists of domain 2 of VEGFR-1 and domain 3 of VEGFR-2 fused with the Fc fragment of IgG1. Aflibercept is commercially marketed under the trade name EYLEA^{®} (aflibercept), which is an ophthalmic intravitreal aflibercept fusion protein injection.

### SUMMARY OF THE DISCLOSURE

While EYLEA^{®} (aflibercept) is the current standard of care for treating wet AMD in patients, a gene therapy method of delivering aflibercept into an eye can provide an improved treatment option for patients because gene therapy can provide prolonged or sustained release of aflibercept in vivo without requiring repeated injections, which can increase the risks of inflammation, infection, and other adverse effects in some patients. Additionally, by not requiring repeated injections, gene therapy addresses the patient compliance and adherence challenge associated with therapies that require repeated injections, as non-compliance can result in vision loss and deterioration of the eye disease or condition. The rate of non-compliance and non-adherence to treatment regimens that require repeated or frequent trips to medical offices for administration is higher among elderly patients, who are most impacted by AMD. Delivering aflibercept into an eye of a patient via gene therapy can thus provide a safer, potentially more cost-effective, and more convenient treatment option for patients, and improve patient outcomes by addressing the non-compliance and non-adherence problem.

The present disclosure relates to pharmaceutical compositions and methods for the prevention or treatment of neovascular (wet) age-related macular degeneration (AMD), macular edema following retinal vein occlusion (RVO), diabetic macular edema (DME), diabetic retinopathy (DR) in patients with DME, retinal vein occlusion, and related eye diseases or conditions, in a primate or human subject by administering intravitreally or subretinally a pharmaceutical composition comprising a pharmaceutically effective amount of a vector or viral particles (e.g., rAAV) comprising a nucleic acid encoding aflibercept, a functional fragment, or a variant thereof.

In some aspects, disclosed herein is a method of treating an eye condition or disease, the method comprising administering a unit dose of a pharmaceutical composition by intravitreal injection to an eye of a primate subject in need thereof, wherein the pharmaceutical composition comprises: (a) a rAAV2 variant comprising an amino acid sequence LGETTRP inserted between positions 587 and 588 of capsid protein VP1, and a nucleic acid sequence encoding a polypeptide having at least 80% homology to aflibercept, a functional fragment, or variant thereof, and (b) a pharmaceutically acceptable excipient. In some cases, the eye condition or disease is neovascular (wet) age-related macular degeneration (AMD), macular edema following retinal vein occlusion, diabetic macular edema (DME), or diabetic retinopathy associated with DME. In some cases, the eye condition or disease is choroidal neovascularization or wet AMD. In some cases, the unit dose comprises between 1E12 to 1E13 vector genomes. In some cases, the unit dose comprises between 2E12 to 6E12 vector genomes. In some cases, the unit dose comprises a volume that is not more than 100 µL. In some cases, the unit dose comprises a volume that is not more than 50 µL. In some cases, the subject is a non-human primate. In some cases, the subject is a human. In some cases, the subject is responsive to aflibercept. In some cases, the subject has been pre-treated with aflibercept. In some cases, the administering by intravitreal injection occurs not more than once in at least 2 years. In some cases, the administering by intravitreal injection occurs not more than once in at least 5 years. In some cases, the administering by intravitreal injection is a one-time administration. In some cases, the pharmaceutical composition is a suspension or a refrigerated suspension. In some cases, the method further comprises agitating the suspension to ensure even distribution of the suspension prior to the administering step. In some cases, the method further comprises warming the pharmaceutical composition to room temperature prior to the administering step.

In other aspects, a pharmaceutical composition comprising a suspension that further comprises a rAAV2 variant comprising an amino acid sequence LGETTRP inserted between positions 587 and 588 of a capsid protein VP1, and a nucleic acid sequence encoding a polypeptide having at least 80% homology to aflibercept, a functional fragment, or variant thereof. In some cases, the nucleic acid sequence comprises a sequence of SEQ ID NO: 2. In some cases, a unit dose of the composition comprises between 1E12 to 1E13 vector genomes. In some cases, a unit dose of the pharmaceutical composition comprises 2E12 to 6E12 vector genomes. In some cases, a kit comprises the suspension pharmaceutical composition and a solution for diluting the pharmaceutical composition. In some cases, the pharmaceutical composition comprises not more than 1 mL. In some cases, the pharmaceutical composition comprises not more than 0.5-1.0 mL. In some cases, the pharmaceutical composition comprises less than or equal to 0.5 mL. In some cases, the solution in the kit comprises a buffer, salt, alcohol, a surfactant, or any combination thereof. In some cases, the kit comprises a syringe.

In other aspects, a method of treating an eye condition or disease comprises: agitating a refrigerated suspension, the suspension comprising: a rAAV2 variant comprising an amino acid sequence LGETTRP inserted between positions 587 and 588 of a capsid protein VP1, and a nucleic acid sequence encoding a polypeptide having at least 80% homology to aflibercept, a functional fragment, or variant thereof; and administering a volume of the refrigerated suspension to an eye of a human subject via intravitreal injection. In some cases, the subject is characterized as having been pre-treated with aflibercept. In some cases, the subject is responsive to aflibercept. In some cases, the refrigerated suspension comprises no more than 1 mL of the solution. In some cases, the refrigerated suspension comprises no more than 0.5 mL. In some cases, the volume administered to the subject is not more than 50 µL. In some cases, the volume administered to the subject is not more than 100 µL. In some cases, the volume comprises a unit dose of between 1E12 to 1E13 vector genomes. In some cases, the volume comprises a unit dose of between 2E12 to 6E12 vector genomes. In some cases, the administering step occurs not more than once in at least 2 years. In some cases, the administering step is a one-time injection. In some cases, the method further comprises assaying the subject for responsiveness to aflibercept before administering the composition. In some cases, the suspension comprises a pharmaceutically acceptable excipient. In some cases, the excipient comprises a surfactant or a stabilizer. In some cases, the surfactant is selected from polysorbates, sodium dodecyl sulfate, sodium lauryl sulfate, lauryl dimethyl amine oxide, polyethoxylated alcohols, polyoxyethylene sorbitan, octoxynol, Brij, pluronic, and polyoxyl castor oil. In some cases, the pharmaceutically acceptable excipient comprises phenol, mannitol, sorbitol, or sodium chloride. In some cases, the eye condition or disease is neovascular (wet) age-related macular degeneration (AMD), macular edema following retinal vein occlusion, diabetic macular edema (DME), or diabetic retinopathy associated with DME. In some cases, the eye condition or disease is choroidal neovascularization or wet AMD. In some cases, the method further comprises warming the suspension to room temperature before administration.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

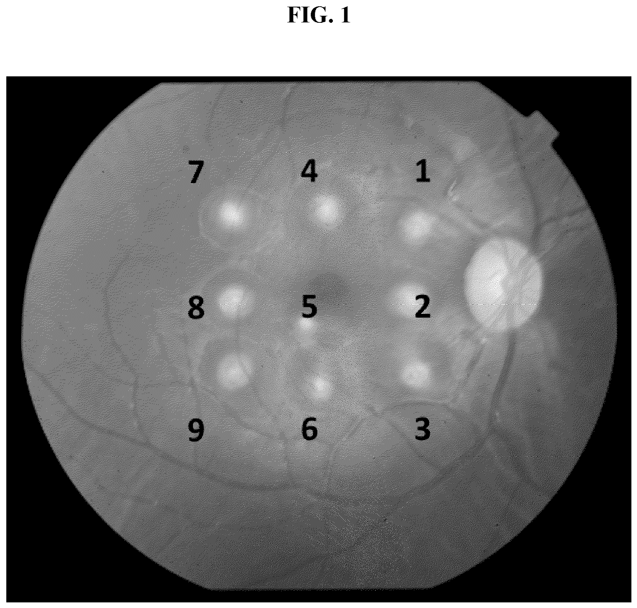

**FIG. 1** illustrates an exemplary fundus image of an eye of a primate (African green monkey) after induction of CNV lesions by laser irradiation without treatment. Nine lesions were induced by single laser application using laser irradiation of 750 mW, 50 µm, 100 ms for all spots except the central spot, which was treated with 400 mW. Color fundus photography was performed immediately after the laser irradiation to document the laser lesions.

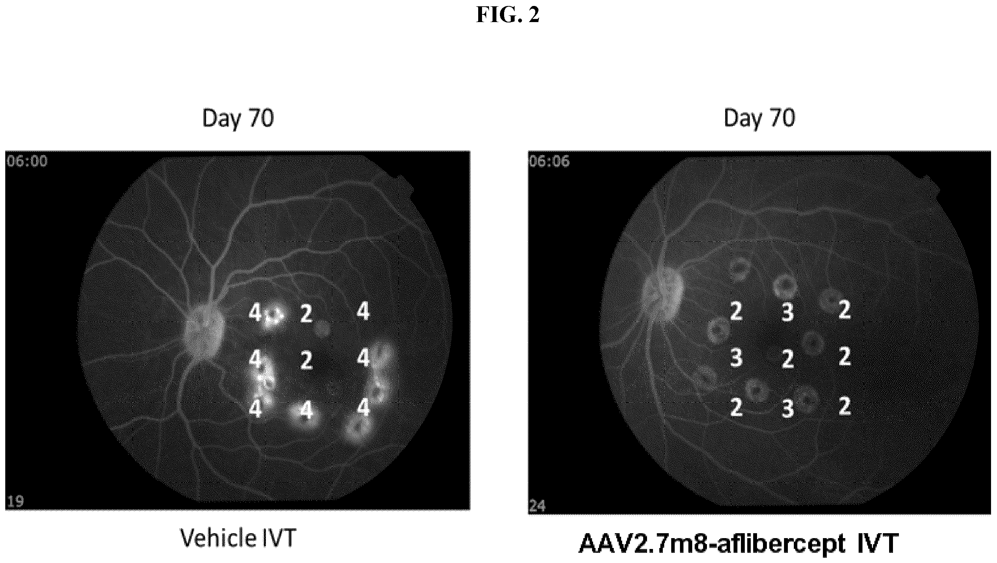

**FIG. 2** illustrates representative fundus images of fluorescence angiography at day 70 after intravitreal (IVT) injection with either vehicle control comprising buffer only or AAV2.7m8-aflibercept. Monkeys treated with AAV2.7m8-aflibercept showered fewer grade IV lesions and lower graded lesions than monkeys injected with the vehicle control only.

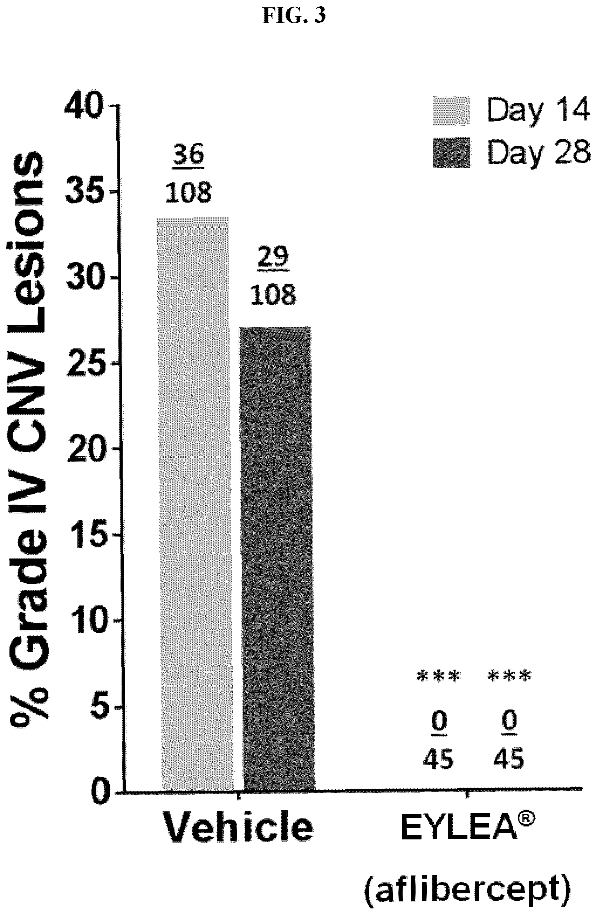

**FIG. 3** illustrates the percent grade IV CNV lesions in monkeys after intravitreal injection of EYLEA^{®} (aflibercept), which was used as a positive control, as compared to vehicle control comprising buffer only. EYLEA^{®} (aflibercept) treatment showed a significant decrease in the amount of grade IV lesions as compared to vehicle control based on fundus images collected at day 14 (light gray bar) and at day 28 (dark gray bar).

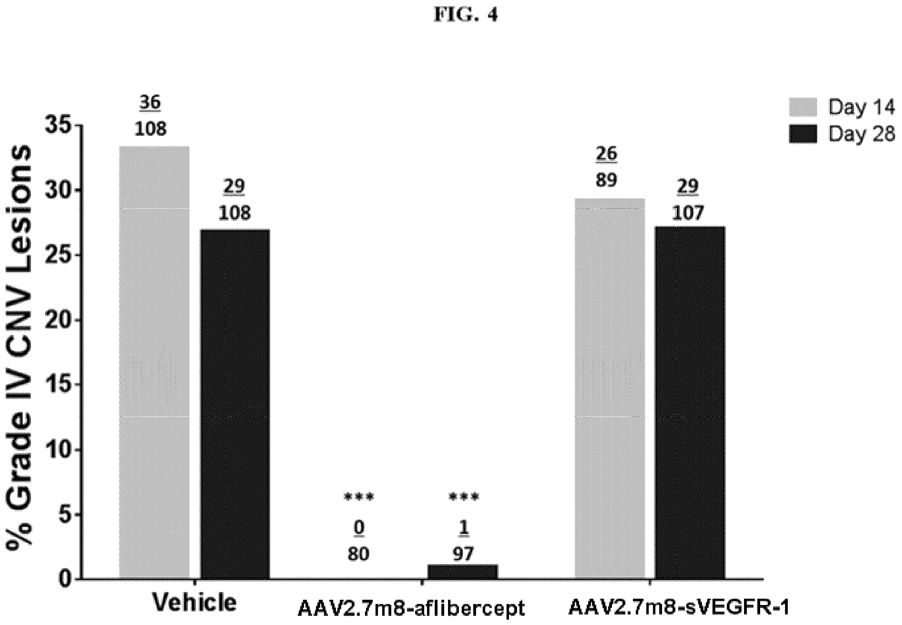

**FIG. 4** illustrates the percent grade IV CNV lesions in monkeys treated with intravitreal injection of vehicle control, AA V2.7m8-aflibercept, or AAV2.7m8-sVEGFR-1 at day 14 (light gray bar) and day 28 (dark gray bar) post intravitreal injection. Monkeys treated with intravitreal AAV2.7m8-aflibercept injection showed a significant decrease in the amount of grade IV lesions as compared to monkeys treated with vehicle control at day 14 and at day 28, similar to the results of the positive control illustrated in **FIG. 3****.** Treatment with intravitreal injection of AAV2.7m8-sVEGFR-1 did not show any significant reduction in grade IV lesions as compared to the vehicle control.

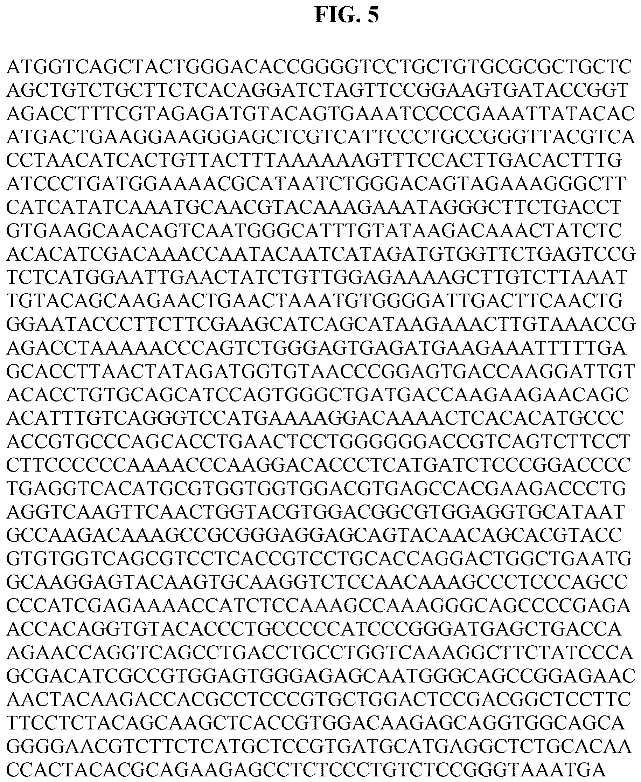

**FIG. 5** illustrates the nucleic acid sequence of aflibercept.

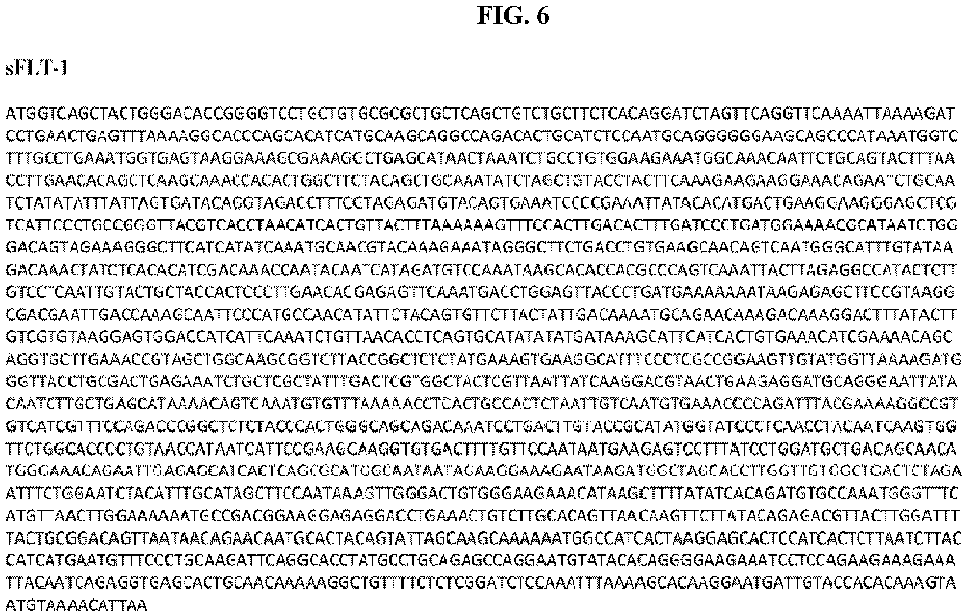

**FIG. 6** illustrates the nucleic acid sequence of soluble fms-like tyrosine kinase-1 (sFlt-1 or sVEGFR-1). sVEGFR-1 is a splice variant of VEGF receptor 1.

### DETAILED DESCRIPTION OF THE DISCLOSURE

Several aspects are described below with reference to example applications for illustration. It should be understood that numerous specific details, relationships, and methods are set forth to provide a full understanding of the features described herein. One having ordinary skill in the relevant art, however, will readily recognize that the features described herein can be practiced without one or more of the specific details or with other methods. The features described herein are not limited by the illustrated ordering of acts or events, as some acts can occur in different orders and/or concurrently with other acts or events. Furthermore, not all illustrated acts or events are required to implement a methodology in accordance with the features described herein.

The present disclosure relates to pharmaceutical compositions and methods of treatment or prevention of eye diseases or conditions comprising administering a gene therapy, a vector, or a construct by intravitreal injection into an eye of a primate (e.g., a monkey or a human) comprising a nucleic acid sequence (e.g., cDNA) that encodes aflibercept, a functional fragment, or a variant thereof. Upon intravitreal injection of a gene therapy, a vector, or a construct comprising the nucleic acid sequence of aflibercept, a functional fragment or variant thereof, the nucleic acid sequence is expressed in vivo, e.g., in retinal cells, to generate the aflibercept fusion protein, or a functional fragment, or variant thereof, which produces a therapeutic effect.

In some embodiments, a gene therapy, vector, or construct comprising aflibercept is used to treat or prevent one or more eye diseases or conditions, including, but not limited to, neovascular (wet) age-related macular degeneration (AMD), macular edema following retinal vein occlusion (RVO), diabetic macular edema (DME), and/or diabetic retinopathy (DR) in patients with DME, retinal vein occlusion, or any other related eye disease or condition involving neovascularization (e.g., choroidal neovascularization (CNV)) in a primate or human subject. In some embodiments, methods described herein are used to treat an eye disease or condition that is responsive to aflibercept (e.g., EYLEA^{®}). In some embodiments, methods described herein are used to treat an eye disease or condition that is responsive to current standard of care or is responsive to at least one of the approved therapies for AMD, RVO, DME, or DR in patients with DME, such as aflibercept injection, ranibizumab injection, or bevacizumab injection.

In some embodiments, by administering intravitreally or subretinally a pharmaceutical composition adapted for or suitable for gene therapy, e.g., a recombinant adeno-associated virus (rAAV) with a variant or modified capsid protein VP1, such as the 7m8 capsid variant, comprising a nucleic acid sequence that encodes a protein or polypeptide that is at least 80%, 85%, 90%, 95%, 96%, 97%, 98%, or 99% homologous to aflibercept, or a functional fragment or variant thereof. Such homology can be based on the nucleic acid sequence, amino acid sequence, spatial conformation, or protein structure of aflibercept.

The protein sequence of aflibercept is publicly available at DrugBank database, accession number DB08885. In some embodiments, aflibercept refers to a nucleic acid sequence that encodes the fusion protein, as disclosed in U.S. Patent Pub. 2014/0371438 **(****FIG. 5****).**

One advantage of gene therapy over protein injections is that gene therapy provides for prolonged or continued release of a therapeutic agent (e.g., aflibercept) and, in some embodiments, does not require multiple or repeated injections. This prolonged or sustained release of aflibercept results from the delivery of a nucleic acid sequence that encodes the aflibercept fusion protein, which is expressed in vivo to provide a therapeutic effect. In some embodiments, expression of aflibercept from the heterologous nucleic acid delivered in retinal cells can continue over at least 1 year, over more than 1 year, over at least 2, 3, 4, 5, 10, or more years.

In some embodiments, a rAAV can comprise a capsid variant protein that increases its infectivity of the target cells or tissue in an eye (e.g., retinal cells), allowing more efficient delivery of the nucleic acid sequence encoding a therapeutic transgene such as aflibercept fusion protein, or a functional fragment or variant thereof, into the target cells or tissue where the therapeutic transgene can be expressed over a period of time (e.g., over at least 1, 1.5, 2, 3, 4, 5, 10 or more years). Gene therapy as disclosed herein can target a specific tissue or cell type of interest, e.g., photoreceptor cells, which can help to minimize off-target effects, or provide a more targeted delivery of the therapeutic transgene such as aflibercept in vivo.

With prolonged or sustained delivery of aflibercept in vivo via gene therapy, one would be able to administer the pharmaceutical composition comprising a nucleic acid sequence that encodes aflibercept, a functional fragment, a mutant or variant thereof, in fewer doses within a period of time as compared to the current standard of care (e.g., protein injections or non-gene therapy-based treatments). In some embodiments, the total number of doses administered of a gene therapy comprising a nucleic acid sequence encoding aflibercept, a functional fragment or variant thereof, is not more than 1 unit dose in at least 1.5 years, at least 2 years, at least 3 years, at least 4 years, at least 5 years, at least 6 years, at least 7 years, at least 8 years, at least 9 years, or at least 10 years. In some embodiments, administration of a gene therapy comprising a nucleic acid sequence encoding aflibercept, a functional fragment or variant thereof, is only one time or once in the lifetime of a patient. In some embodiments, one-time administration of a gene therapy comprising a nucleic acid sequence encoding aflibercept, a functional fragment or variant thereof, can produce a therapeutic effect in a patient that lasts for more than 1 year, or more than 2, 3, 4, 5, 6, 7, 8, 9, or 10 years. In some embodiments, a gene therapy comprising a nucleic acid sequence encoding aflibercept, a functional fragment or variant thereof, is administered not more than once to a patient in at least 2 or more years, in at least 3 or more years, in at least 4 or more years, in at least 5 or more years, in at least 6 or more years, in at least 7 or more years, in at least 8 or more years, in at least 9 or more years, or in at least 10 or more years. In some embodiments, a gene therapy comprising a nucleic acid sequence encoding aflibercept is administered to a patient who is responsive to aflibercept or who received a pre-treatment with EYLEA^{®} before receiving the gene therapy disclosed herein. In some embodiments, a patient who receives the gene therapy disclosed herein (e.g., AAV2.7m8-aflibercept) can commence therapy with aflibercept, ranibizumab, and/or bevacizumab, as needed, after at least 2, 3, 4, 5, 10 or more years have lapsed after receiving the gene therapy. In some embodiments, anti-VEGF therapy is not needed after a patient receives a treatment with the gene therapy disclosed herein.

In some embodiments, the one-time administration of a gene therapy comprising a nucleic acid sequence encoding aflibercept, a functional fragment or variant thereof, obviates the need for the patient to receive an EYLEA^{®} injection for more than a year, for more than 1.5 years, or for more than 2, 3, 4, 5, 6, 7, 8, 9, 10 years. In some embodiments, a patient who receives an intravitreal injection of a gene therapy comprising a nucleic acid sequence encoding aflibercept, a functional fragment or variant thereof, does not need any additional injection of aflibercept for the remainder of the patient's life. In other embodiments, a patient who receives a one-time intravitreal injection of 7m8-aflibercept gene therapy can commence therapy with any one of aflibercept, ranibizumab, and/or bevacizumab, as needed, at least 1.5, 2, 3, 4, 5, 6, 7, 8, 9, 10 or more years after receiving the gene therapy.

The terminology of the present disclosure is for the purpose of describing particular cases only and is not intended to be limiting of compositions, methods and compositions of this disclosure.

The compositions and methods of this disclosure as described herein may employ, unless otherwise indicated, conventional techniques and descriptions of molecular biology (including recombinant techniques), cell biology, biochemistry, immunochemistry and ophthalmic techniques, which are within the skill of those who practice in the art. Such conventional techniques include methods for observing and analyzing the retina, or vision in a subject, cloning and propagation of recombinant virus, formulation of a pharmaceutical composition, and biochemical purification and immunochemistry. Specific illustrations of suitable techniques can be had by reference to the examples herein. However, equivalent conventional procedures can, of course, also be used. Such conventional techniques and descriptions can be found in standard laboratory manuals such as Green, et al., Eds., Genome Analysis: A Laboratory Manual Series (Vols. I-IV) (1999); Weiner, et al., Eds., Genetic Variation: A Laboratory Manual (2007); Dieffenbach, Dveksler, Eds., PCR Primer: A Laboratory Manual (2003); Bowtell and Sambrook, DNA Microarrays: A Molecular Cloning Manual (2003); Mount, Bioinformatics: Sequence and Genome Analysis (2004); Sambrook and Russell, Condensed Protocols from Molecular Cloning: A Laboratory Manual (2006); and Sambrook and Russell, Molecular Cloning: A Laboratory Manual (2002) (all from Cold Spring Harbor Laboratory Press); Stryer, L., Biochemistry (4th Ed.) W.H. Freeman, N.Y. (1995); Gait, "Oligonucleotide Synthesis: A Practical Approach" IRL Press, London (1984); Nelson and Cox, Lehninger, Principles of Biochemistry, 3rd Ed., W.H. Freeman Pub., New York (2000); and Berg et al., Biochemistry, 5th Ed., W.H. Freeman Pub., New York (2002), all of which are herein incorporated by reference in their entirety for all purposes.

In some embodiments, disclosed herein are pharmaceutical formulations comprising: (a) a recombinant adeno-associated virus (rAAV2) virion adapted for gene therapy comprising: (i) a variant AAV2 capsid protein, wherein the variant AAV2 capsid protein comprises LGETTRP insertion between positions 587 and 588, and wherein the variant capsid protein confers an increase in an infectivity of an ocular cell relative to an AAV virion that comprises a corresponding non-variant AAV2 capsid protein; and (ii) a heterologous nucleic acid sequence encoding aflibercept, a functional fragment or variant thereof; and (b) a pharmaceutically acceptable excipient. In some embodiments, the gene product that is encoded is a fusion protein or a polypeptide having at least 80%, 85%, 90%, 95%, or 99% homology to aflibercept.

Also disclosed herein are methods of treating an eye condition or disease for which aflibercept is indicated or approved for treating, comprising administering a pharmaceutical formulation adapted for gene therapy, i.e., delivering a nucleic acid sequence that encodes aflibercept in vivo, as described herein, to an eye of a subject by intravitreal injection.

Also disclosed herein are pharmaceutical compositions comprising a gene therapy or a vector that encodes a fusion protein or polypeptide having at least 80%, 85%, 90%, 95%, or 99% homology to aflibercept that can be lyophilized, or supplied in suspension form. In some embodiments, a lyophilized form or a suspension of the pharmaceutical composition is provided in a kit with a buffer for reconstituting the pharmaceutical composition or for dilution, respectively.

Also disclosed herein are pharmaceutical compositions comprising a gene therapy or a vector that encodes a fusion protein or polypeptide having at least 80%, 85%, 90%, 95%, or 99% homology to aflibercept that is supplied as a refrigerated suspension. In some embodiments, a refrigerated suspension of the pharmaceutical composition is provided in a kit with a buffer for diluting the pharmaceutical composition.

Also disclosed herein are recombinant adeno-associated virus (rAAV) virions adapted for gene therapy for reducing choroidal neovascularization comprising: (a) a variant AAV2 capsid protein comprising a peptide insertion of amino acid sequence LGETTRP inserted at amino acid position between 587 and 588 in AAV2, wherein the peptide insertion confers an increase in an infectivity of an ocular cell relative to an AAV virion that comprises a corresponding non-variant or unmodified AAV2 capsid protein; (b) a heterologous nucleic acid sequence encoding a polypeptide or therapeutic transgene having at least 80% homology to aflibercept, or a functional fragment or variant thereof.

Also disclosed herein are methods of treating an eye condition or disease comprising administering a rAAV virion adapted for gene therapy and in vivo delivery of a nucleic acid sequence for expressing aflibercept, or a functional fragment or variant thereof, as described herein to an eye of a human subject; where the human subject has been previously diagnosed with an eye condition associated with neovascularization. In some embodiments, the aflibercept gene therapy is administered to a patient who is responsive to aflibercept or who was pre-treated with EYLEA^{®} (aflibercept).

In some embodiments, disclosed herein are methods and pharmaceutical formulations comprising: (a) a recombinant adeno-associated virus (rAAV) virion adapted for gene therapy comprising: (i) a variant AAV capsid protein comprising an amino acid insertion selected from LGETTRP, NETITRP, KAGQANN, KDPKTTN, KDTDTTR, RAGGSVG, AVDTTKF, and STGKVPN at a position that corresponds to amino acids 570-611 of capsid protein VP1 in AAV2, and where the variant capsid protein confers an increase in an infectivity of a retinal cell (e.g., photoreceptor cells or retinal pigment epithelium) relative to an AAV virion that comprises a corresponding non-variant AAV2 capsid protein; and (ii) a heterologous nucleic acid sequence encoding aflibercept; and (b) a pharmaceutically acceptable excipient. In some embodiments, the gene product that is encoded is a fusion protein or a polypeptide having at least 80% homology to aflibercept, or a functional fragment or variant thereof. In some embodiments, a pharmaceutically acceptable excipient comprises a surfactant (e.g., non-ionic surfactant, pluronic, poloxamer, or polysorbate) that prevents aggregation in the pharmaceutical composition disclosed herein.

Unless otherwise defined, all technical terms used herein have the same meaning as commonly understood by one of ordinary skill in the art.

The terminology used herein is for the purpose of describing particular cases only and is not intended to be limiting. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising". The term "comprising" as used herein is synonymous with "including" or "containing", and is inclusive or open-ended.

Any reference to "or" herein is intended to encompass "and/or" unless otherwise stated. As used herein, the term "about" a number refers to that number plus or minus 10% of that number. The term "about" a range refers to that range minus 10% of its lowest value and plus 10% of its greatest value.

The term "subject", "patient", or "individual" refers to primates, including non-human primates such as monkeys, e.g., African green monkeys and rhesus monkeys, and humans. In preferred embodiments, the subject is a human or a human patient.

The terms "treat," "treating", "treatment," "ameliorate" or "ameliorating" and other grammatical equivalents as used herein, include alleviating, abating or ameliorating a disease or condition symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition, and are intended to include prophylaxis. The terms further include achieving a therapeutic benefit and/or a prophylactic benefit. By therapeutic benefit is meant eradication or amelioration of the underlying disease being treated. Also, a therapeutic benefit is achieved with the eradication or amelioration of one or more of the physiological symptoms associated with the underlying disease such that an improvement is observed in the patient, notwithstanding that, in some embodiments, the patient is still afflicted with the underlying disease. For prophylactic benefit, the pharmaceutical compositions are administered to a patient at risk of developing a particular disease, or to a patient reporting one or more of the physiological symptoms of a disease, even if a diagnosis of the disease has not been made.

The terms "administer," "administering", "administration," and the like, as used herein, can refer to the methods that are used to enable delivery of therapeutics or pharmaceutical compositions to the desired site of biological action. These methods include intravitreal or subretinal injection to an eye.

The terms "effective amount", "therapeutically effective amount" or "pharmaceutically effective amount" as used herein, can refer to a sufficient amount of at least one pharmaceutical composition or compound being administered which will relieve to some extent one or more of the symptoms of the disease or condition being treated.

The term "pharmaceutically acceptable" as used herein, can refer to a material, such as a carrier or diluent, which does not abrogate the biological activity or properties of a compound disclosed herein, and is relatively nontoxic (i.e., when the material is administered to an individual it does not cause undesirable biological effects nor does it interact in a deleterious manner with any of the components of the composition in which it is contained).

The term "pharmaceutical composition," or simply "composition" as used herein, can refer to a biologically active compound, optionally mixed with at least one pharmaceutically acceptable chemical component, such as, though not limited to carriers, stabilizers, diluents, dispersing agents, suspending agents, thickening agents, excipients and the like.

An "AAV vector" or "rAAV vector" as used herein refers to an adeno-associated virus (AAV) vector or a recombinant AAV (rAAV) vector comprising a polynucleotide sequence not of AAV origin (i.e., a polynucleotide heterologous to AAV such as a nucleic acid sequence that encodes a therapeutic transgene, e.g., aflibercept), typically a sequence of interest for the genetic transformation of a cell. In general, the heterologous polynucleotide is flanked by at least one, and generally by two, AAV inverted terminal repeat sequences (ITRs). The term rAAV vector encompasses both rAAV vector particles and rAAV vector plasmids. A rAAV vector may either be single-stranded (ssAAV) or self-complementary (scAAV).

An "AAV virus" or "AAV viral particle" or "rAAV vector particle" refers to a viral particle composed of at least one AAV capsid protein (typically by all of the capsid proteins of a wild-type AAV) and a polynucleotide rAAV vector. If the particle comprises a heterologous polynucleotide (i.e. a polynucleotide other than a wild-type AAV genome such as a transgene to be delivered to a mammalian cell), it is typically referred to as a "rAAV vector particle" or simply an "rAAV vector". Thus, production of rAAV particle necessarily includes production of rAAV vector, as such a vector is contained within a rAAV particle..

The term "packaging" as used herein can refer to a series of intracellular events that can result in the assembly and encapsidation of a rAAV particle.

AAV "rep" and "cap" genes refer to polynucleotide sequences encoding replication and encapsidation proteins of adeno-associated virus. AAV rep and cap are referred to herein as AAV "packaging genes."

The term "polypeptide" can encompass both naturally-occurring and non-naturally occurring proteins (e.g., a fusion protein), peptides, fragments, mutants, derivatives and analogs thereof. A polypeptide may be monomeric, dimeric, trimeric, or polymeric. Further, a polypeptide may comprise a number of different domains each of which has one or more distinct activities. For the avoidance of doubt, a "polypeptide" may be any length greater two amino acids.

As used herein, "polypeptide variant" or simply "variant" refers to a polypeptide whose sequence contains an amino acid modification. In some instances, the modification can be an insertion, duplication, deletion, rearrangement or substitution of one or more amino acids compared to the amino acid sequence of a reference protein or polypeptide, such as a native or wild-type protein. A variant may have one or more amino acid point substitutions, in which a single amino acid at a position has been changed to another amino acid, one or more insertions and/or deletions, in which one or more amino acids are inserted or deleted, respectively, in the sequence of the reference protein, and/or truncations of the amino acid sequence at either or both the amino or carboxy termini. A variant can have the same or a different biological activity compared to the reference protein, or the unmodified protein.

In some embodiments, a variant can have, for example, at least about 80%, 85%, 90%, 91%, 92%, 93%, 94%, 95%, 96%, 97%, 98%, 99% overall sequence homology to its counterpart reference protein. In some embodiments, a variant can have at least about 90% overall sequence homology to the wild-type protein. In some embodiments, a variant exhibits at least about 95%, at least about 98%, at least about 99%, at least about 99.5%, or at least about 99.9% overall sequence identity.

As used herein, "recombinant" can refer to a biomolecule, e.g., a gene or protein, that (1) has been removed from its naturally occurring environment, (2) is not associated with all or a portion of a polynucleotide in which the gene is found in nature, (3) is operatively linked to a polynucleotide which it is not linked to in nature, or (4) does not occur in nature. The term "recombinant" can be used in reference to cloned DNA isolates, chemically synthesized polynucleotide analogs, or polynucleotide analogs that are biologically synthesized by heterologous systems, as well as proteins and/or mRNAs encoded by such nucleic acids. Thus, for example, a protein synthesized by a microorganism is recombinant, for example, if it is synthesized from an mRNA synthesized from a recombinant gene present in the cell.

"Operatively linked" or "operably linked" or "coupled" can refer to a juxtaposition of genetic elements, wherein the elements are in a relationship permitting them to operate in an expected manner. For instance, a promoter can be operatively linked to a coding region if the promoter helps initiate transcription of the coding sequence. There may be intervening residues between the promoter and coding region so long as this functional relationship is maintained.

The term "expression vector" or "expression construct" or "cassette" or "plasmid" or simply "vector" can include any type of genetic construct, including AAV or rAAV vectors, containing a nucleic acid or polynucleotide coding for a gene product in which part or all of the nucleic acid encoding sequence is capable of being transcribed and is adapted for gene therapy. The transcript can be translated into a protein. In some cases, it may be partially translated or not translated. In certain aspects, expression includes both transcription of a gene and translation of mRNA into a gene product. In other aspects, expression only includes transcription of the nucleic acid encoding genes of interest. An expression vector can also comprise control elements operatively linked to the encoding region to facilitate expression of the protein in target cells. The combination of control elements and a gene or genes to which they are operably linked for expression can sometimes be referred to as an "expression cassette," a large number of which are known and available in the art or can be readily constructed from components that are available in the art.

The term "heterologous" can refer to an entity that is genotypically distinct from that of the rest of the entity to which it is being compared. For example, a polynucleotide introduced by genetic engineering techniques into a plasmid or vector derived from a different species can be a heterologous polynucleotide. A promoter removed from its native coding sequence and operatively linked to a coding sequence with which it is not naturally found linked can be a heterologous promoter.

As used herein, "7m8" refers to the 7-mer amino acid sequence LGETTRP.

"7m8 variant" refers to a rAAV, which can be of any serotype, with the amino acid sequence LGETTRP inserted in the solvent exposed GH loop of the capsid protein.

When 7m8 is inserted in a rAAV2 (also referred to as AAV2.7m8), the 7-mer amino acid sequence LGETTRP is inserted into the GH loop of the AAV2 capsid protein, e.g., between positions 587 and 588 of the AAV2 capsid protein, VP1. When 7m8 is inserted in a rAAV1 (also referred to as AAV1.7m8), the 7-mer amino acid sequence LGETTRP is inserted into the GH loop of the AAV1 capsid protein, e.g., between amino acids 590 and 591 of the AAV1 capsid protein. When 7m8 is inserted in a rAAV5 (also referred to as AAV5.7m8), the 7-mer amino acid sequence LGETTRP is inserted into the GH loop of the AAV5 capsid protein, e.g., between amino acids 575 and 576 of the AAV5 capsid protein. When 7m8 is inserted in a rAAV6 (also referred to as AAV6.7m8), the 7-mer amino acid sequence LGETTRP is inserted into the GH loop of the AAV6 capsid protein, e.g., between amino acids 590 and 591 of the AAV6 capsid protein. When 7m8 is inserted in a rAAV7 (also referred to as AAV7.7m8), the 7-mer amino acid sequence LGETTRP is inserted into the GH loop of the AAV7 capsid protein, e.g., between amino acids 589 and 590 of the AAV7 capsid protein. When 7m8 is inserted in a rAAV8 (also referred to as AAV8.7m8), the 7-mer amino acid sequence LGETTRP is inserted into the GH loop of the AAV8 capsid protein, e.g., between amino acids 590 and 591 of the AAV8 capsid protein. When 7m8 is inserted in a rAAV9 (also referred to as AAV9.7m8), the 7-mer amino acid sequence LGETTRP is inserted into the GH loop of the AAV9 capsid protein, e.g., between amino acids 588 and 589 of the AAV9 capsid protein. When 7m8 is inserted in a rAAV10 (also referred to as AAV10.7m8), the 7-mer amino acid sequence LGETTRP is inserted into the GH loop of the AAV10 capsid protein, e.g., between amino acids 589 and 590 of the AAV10 capsid protein.

The section headings used herein are for organizational purposes only and are not to be construed as limiting the subject matter described.

### Vectors

In some embodiments, pharmaceutical compositions and methods of the disclosure provide for delivery of a nucleic acid sequence (e.g., cDNA sequence) encoding aflibercept, a functional fragment or variant thereof, to retinal cells in a human subject or patient in need thereof (e.g., a patient diagnosed with AMD, RVO, DME). Delivery of the nucleic acid of a therapeutic transgene to a patient using a delivery system, such as rAAV or a viral vector, is also referred to as gene therapy.

In some embodiments, delivery of the aflibercept nucleic acid sequence can be performed using any suitable "vector" (also referred to as "gene delivery" or "gene transfer vehicle"). Vector (e.g., rAAV), delivery vehicle, gene delivery vehicle or gene transfer vehicle, can encompass any suitable macromolecule or complex of molecules comprising a polynucleotide to be delivered to a target cell, e.g., retinal cells, including photoreceptor, a retinal ganglion cell, a Müller cell, a bipolar cell, an amacrine cell, a horizontal cell, or a retinal pigmented epithelium cell. In some cases, a target cell can be any cell to which the nucleic acid molecule or gene is delivered. The polynucleotide to be delivered can comprise a coding sequence of a therapeutic transgene, such as the aflibercept transgene.

The composition and methods of the disclosure provide for any suitable method for delivery of an aflibercept nucleic acid sequence into an eye or retinal cells of a non-human primate or human subject. In some cases, delivery of the nucleic acid molecule, polynucleotide, or gene therapy is formulated or adapted for intravitreal injection into an eye of a non-human primate or human subject.

In some embodiments, suitable vectors include, but are not limited to, viral vectors such as adenoviruses, adeno-associated viruses (AAV), and retroviruses, retrovirus, lentivirus, liposomes, lipid-containing complexes, nanoparticles, and other macromolecular complexes capable of delivery of a polynucleotide to retinal cells. In some embodiments, the viral vector comprises a strong eukaryotic promoter operably linked to the polynucleotide e.g., a cytomegalovirus (CMV) promoter or a constitutive promoter.

In some embodiments, a vector comprises a recombinant viral vector that incorporates one or more nucleic acid molecules. As described herein, nucleic acids refer to polynucleotides. Nucleic acid and polynucleotide may be used interchangeably. In some embodiments, nucleic acids comprise DNA or RNA. In some cases, nucleic acids include DNA (e.g., cDNA) or RNA for the expression of aflibercept. In some embodiments, RNA can include a transcript of a gene of interest (e.g., aflibercept), introns, untranslated regions (UTRs), termination sequences and the like. In other embodiments, DNA can include, but are not limited to, sequences such as promoter sequences, a gene of interest (e.g. aflibercept), UTRs, termination sequences, and the like. In some cases, a combination of DNA and RNA can be used.

In some embodiments, the present disclosure provides a recombinant virus, such as recombinant adeno-associated virus (rAAV) as a vector for delivery and expression of aflibercept in a subject.

In some embodiments, any suitable viral vectors can be engineered or optimized for use with the compositions and methods of the disclosure. For example, recombinant viral vectors derived from adenovirus (Ad) or adeno-associated virus (AAV) can be altered such that it is replication-defective in human or primate subjects. In some embodiments, hybrid viral vector systems can be obtained using methods known to one skilled in the art and used to deliver a nucleic acid encoding aflibercept to retinal cells. In some embodiments, a viral delivery system or gene therapy can integrate a nucleic acid sequence comprising the aflibercept gene into the target cell genome (e.g., genome of retinal cells) and result in stable gene expression of the gene over time. In some embodiments, the aflibercept gene is not integrated into the target cell genome, and is expressed from a plasmid or vector introduced into the target cells.

In some embodiments, a suitable viral vector for delivering a nucleic acid sequence of aflibercept to retinal cells is AAV or rAAV, which are small non-enveloped single-stranded DNA viruses. rAAVs are non-pathogenic human parvoviruses and can be made to be dependent on helper viruses, including adenovirus, herpes simplex virus, vaccinia virus and CMV, for replication. Exposure to wild-type (wt) AAV is not associated or known to cause any human pathologies and is common in the general population, making AAV or rAAV a suitable delivery system for gene therapy. AAV and rAAV used for gene therapy for delivery of a therapeutic transgene, e.g., aflibercept, can be of any serotype. In some embodiments, pharmaceutical compositions and methods of the disclosure provide for use of any suitable AAV serotype, including AAV1, AAV2, AAV2.5, AAV3, AAV4, AAV5, AAV6, AAV7, AAV8, AAV9, AAV10, AAV11, AAV12, rh10, AAV-DJ, and any hybrid or chimeric AAV thereof. In some embodiments, the serotype used is based on tropism of the virus, or infectivity of a target cell of interest. In some embodiments, AAV2 or rAAV2 is used to deliver a nucleic acid sequence encoding aflibercept into an eye or retinal cells of a subject via intravitreal or subretinal injection. In some embodiments, rAAV2.7m8 is used to deliver the nucleic acid sequence of aflibercept into the retinal cells of a subject.

In some embodiments, AAV or rAAV viruses, particles, or virions comprising a variant capsid protein having increased infectivity of target cells, e.g. retinal cells, are used to increase transduction of retinal cells or to increase targeting of gene delivery to retinal cells in a subject. In some embodiments, the rAAV virion comprises an amino acid modification in a capsid protein GH loop/loop IV of the AAV capsid protein. In some cases, the site of modification is a solvent-accessible portion of the GH loop/loop IV of the AAV capsid protein. For a description of the GH loop/loop IV of AAV capsid, see, e.g., van Vliet et al. (2006) Mol. Ther. 14:809; Padron et al. (2005) J. Virol. 79:5047; and Shen et al. (2007) Mol. Ther. 15:1955. Several AAV capsid variants are known, including the 7m8 variant. In some embodiments, a rAAV virion comprises a variant AAV capsid protein that comprises an insertion of from 5 amino acids to 11 amino acids, e.g., 7 amino acid sequence, in the GH loop of a capsid protein relative to a corresponding parental AAV capsid protein, and wherein the variant capsid protein confers increased infectivity of a retinal cell compared to the infectivity of the retinal cell by an AAV virion comprising the corresponding parental or unmodified AAV capsid protein. In some embodiments, any one of the following amino acid sequences can be inserted in the GH loop of a capsid protein: LGETTRP (7m8), NETITRP, KAGQANN, KDPKTTN, KDTDTTR, RAGGSVG, AVDTTKF, and STGKVPN. In some embodiments, any one of amino acid sequences LGETTRP (7m8), NETITRP, KAGQANN, KDPKTTN, KDTDTTR, RAGGSVG, AVDTTKF, and STGKVPN is inserted in the solvent-exposed GH loop of VP1 capsid protein in a rAAV. In some embodiments, rAAV.7m8 comprising aflibercept is used for gene therapy.

In some embodiments, any one of the following amino acid sequences: NETITRP, KAGQANN, KDPKTTN, KDTDTTR, RAGGSVG, AVDTTKF, and STGKVPN can be inserted at the following positions to generate a rAAV variant for use in gene therapy: between positions 587 and 588 of the AAV2 capsid protein; between amino acids 590 and 591 of the AAV1 capsid protein; between amino acids 575 and 576 of the AAV5 capsid protein; between amino acids 590 and 591 of the AAV6 capsid protein; between amino acids 589 and 590 of the AAV7 capsid protein; between amino acids 590 and 591 of the AAV8 capsid protein; between amino acids 588 and 589 of the AAV9 capsid protein; or between amino acids 589 and 590 of the AAV10 capsid protein.

In some embodiments, the nucleic acid encoding a gene product such as aflibercept can be under transcriptional control by a promoter that initiates transcription of the gene. In some embodiments, the promoter is a "strong" or constitutively active promoter, e.g., CMV promoter. In some embodiment, the connexin 36 promoter is used to drive expression of a therapeutic transgene, e.g., aflibercept. In some embodiments, tissue-specific promoters can be used to effect transcription in specific tissues or cells, such as retinal cells, to reduce potential toxicity or undesirable effects to non-targeted cells. In some embodiments, a recombinant virus and/or plasmid used to generate a rAAV virus can comprise other transcriptional or regulatory elements, such as poly A (polyadenylation) sequence, untranslated regions (UTRs), 3' UTRs, or termination sequences. In some embodiments, more than one genes can be expressed from the vector or plasmid using internal ribosome entry site (IRES) or similar elements that allow co-expression of two or more proteins or create multigene, or polycistronic mRNA.

In some embodiments, the rAAV and/or plasmid used to generate rAAV viruses comprises the following nucleic acid elements: a first ITR sequence; a promoter sequence; an intron sequence; a first UTR sequence; a sequence encoding aflibercept; a second UTR sequence; a polyA sequence; and a second ITR sequence. In some embodiments, a linker sequence is used between each of these nucleic acid elements. In some embodiments, the sequence encoding aflibercept comprises a sequence encoding the aflibercept fusion protein or a functional fragment thereof.

In some embodiments, the viral vector of the disclosure is measured as vector genomes. In some cases, a unit dose of recombinant viruses of this disclosure comprises between 1×10¹⁰ to 2×10¹⁰, between 2×10¹⁰ to 3×10¹⁰, between 3×10¹⁰ to 4×10¹⁰, between 4×10¹⁰ to 5×10¹⁰, between 5×10¹⁰ to 6×10¹⁰, between 6×10¹⁰ to 7×10¹⁰, between 7×10¹⁰ to 8×10¹⁰, between 8×10¹⁰ to 9×10¹⁰, between 9×10¹⁰ to 10×10¹⁰, between 1×10¹¹ to 2×10¹¹, between 2×10¹¹ to 3×10¹¹, between 3×10¹¹ to 4×10¹¹, between 4×10¹¹ to 5×10¹¹, between 5×10¹¹ to 6×10¹¹, between 6×10¹¹ to 7×10¹¹, between 7×10¹¹ to 8×10¹¹, between 8×10¹¹ to 9×10¹¹, between 9×10¹¹ to 10×10¹¹, between 1×10¹² to 2×10¹², between 2×10¹² to 3×10¹², between 3×10¹² to 4×10¹², between 4×10¹² to 5×10¹², between 5×10¹² to 6×10¹², between 6×10¹² to 7×10¹², between 7×10¹² to 8×10¹², between 8×10¹² to 9×10¹², between 9×10¹² to 10×10¹², between 1×10¹³ to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 9×10¹³ to 10×10¹³ vector genomes. In some embodiments, the rAAV of this disclosure is about 2.1×10¹² vector genomes. In some embodiments, the rAAV of the disclosure comprises 2E12 to 6E12 vector genomes. In some embodiments, the rAAV of this disclosure is between 10¹⁰ to 10¹³, between 10¹⁰ to 10¹¹, between 10¹¹ to 10¹², between 10¹² to 10¹³, between 10¹³ to 10¹⁴, between 2×10¹¹ to 4×10¹¹, between 3×10¹¹ to 5×10¹¹, between 4×10¹¹ to 6×10¹¹, between 5×10¹¹ to 7×10¹¹, between 6×10¹¹ to 8×10¹¹, between 7×10¹¹ to 9×10¹¹, between 8×10¹¹ to 10×10¹¹, between 1×10¹² to 3×10¹², between 2×10¹² to 4×10¹², between 3×10¹² to 5×10¹², between 4×10¹² to 6×10¹², between 5×10¹² to 7×10¹², between 6×10¹² to 8×10¹², between 7×10¹² to 9×10¹², between 8×10¹² to 10×10¹², between 1×10¹³ to 5×10¹³, between 5×10¹³ to 10×10¹³, between 10¹² to 5×10¹², or between 5×10¹² to 1×10¹³ vector genomes.

In some embodiments, a lower amount or range of vector genomes is selected for a unit dose to avoid aggregation. In some embodiments, a higher amount or range of vector genomes is selected for a unit dose so that a smaller volume can be used for injection. Smaller volume (e.g., less than 50, 40, 30, 20, 10, or 5 µL) of injection can help to reduce changes in ocular pressure and other adverse effects associated with intravitreal injection. In some embodiments, a higher concentration of rAAV also helps to ensure efficient delivery of the therapeutic transgene into target cells.

In some cases, recombinant viruses of this disclosure are about 1E10, about 1.5E10, about 2E10, about 2.5E10, about 3E10, about 3.5E10, about 4E10, about 4.5E10, about 5E10, about 5.5E10, about 6E10, about 6.5E10, about 7E10, about 7.5E10, about 8E10, about 8.5E10, about 9E10, about 9.5E10, about 10E10, about 1E11, about 1.5E11, about 2E11, about 2.5E11, about 3E11, about 3.5E11, about 4E11, about 4.5E11, about 5E11, about 5.5E11, about 6E11, about 6.5E11, about 7E11, about 7.5E11, about 8E11, about 8.5E11, about 9E11, about 9.5E11, about 10E11, about 1E12, about 1.3E12, about 1.5E12, about 2E12, about 2.1E12, about 2.3E12, about 2.5E12, about 2.7E12, about 2.9E12, about 3E12, about 3.1E12, about 3.3E12, about 3.5E12, about 3.7E12, about 3.9E12, about 4E12, about 4.1E12, about 4.3E12, about 4.5E12, about 4.7E12, about 4.9E12, about 5E12, about 5.1E12, about 5.3E12, about 5.5E12, about 5.7E12, about 5.9E12, about 6E12, about 6.1E12, about 6.3E12, about 6.5E12, about 6.7E12, about 6.9E12, about 7E12, about 7.1E12, about 7.3E12, about 7.5E12, about 7.7E12, about 7.9E12, about 8E12, about 8.1E12, about 8.3E12, about 8.5E12, about 8.7E12, about 8.9E12, about 9E12, about 9.1E12, about 9.3E12, about 9.5E12, about 9.7E12, about 9.9E12, about 10E12, about 10.1E12, about 10.3E12, about 10.5E12, about 10.7E12, about 10.9E12, about 11E12, about 11.5E12, about 12E12, about 12.5E12, about 13E12, about 13.5E12, about 14E12, about 14.5E12, about 15E12, about 15.5E12, about 16E12, about 16.5E12, about 17E12, about 17.5E12, about 18E12, about 18.5E12, about 19E12, about 19.5E12, about 20E12, about 20.5E12, about 30E12, about 30.5E12, about 40E12, about 40.5E12, about 50E12, about 50.5E12, about 60E12, about 60.5E12, about 70E12, about 70.5E12, about 80E12, about 80.5E12, about 90E12, about 95E12, or about 100E12, wherein E is a short-hand for base 10 for exponentiation, and xEy refers to x multiplied by base 10 to the y power/exponent.

In some embodiments, pharmaceutical compositions disclosed herein comprise recombinant viruses of at least 5E11, at least 5.5E11, at least 6E11, at least 6.5E11, at least 7E11, at least 7.5E11, at least 8E11, at least 8.5E11, at least 9E11, at least 9.5E11, at least 10E11, at least 1E12, at least 1.3E12, at least 1.5E12, at least 2E12, at least 2.1E12, at least 2.3E12, at least 2.5E12, at least 2.7E12, at least 2.9E12, at least 3E12, at least 3.1E12, at least 3.3E12, at least 3.5E12, at least 3.7E12, at least 3.9E12, at least 4E12, at least 4.1E12, at least 4.3E12, at least 4.5E12, at least 4.7E12, at least 4.9E12, at least 5E12, at least 5.1E12, at least 5.3E12, at least 5.5E12, at least 5.7E12, at least 5.9E12, at least 6E12, at least 6.1E12, at least 6.3E12, at least 6.5E12, at least 6.7E12, at least 6.9E12, at least 7E12, at least 7.1E12, at least 7.3E12, at least 7.5E12, at least 7.7E12, at least 7.9E12, at least 8E12, at least 8.1E12, at least 8.3E12, at least 8.5E12, at least 8.7E12, at least 8.9E12, at least 9E12, at least 9.1E12, at least 9.3E12, at least 9.5E12, at least 9.7E12, at least 9.9E12, at least 10E12, at least 10.1E12, at least 10.3E12, at least 10.5E12, at least 10.7E12, at least 10.9E12, at least 11E12, at least 11.5E12, at least 12E12, at least 12.5E12, at least 13E12, at least 13.5E12, at least 14E12, at least 14.5E12, at least 15E12, at least 15.5E12, at least 16E12, at least 16.5E12, at least 17E12, at least 17.5E12, at least 18E12, at least 18.5E12, at least 19E12, at least 19.5E12, at least 20E12, at least 20.5E12, at least 30E12, at least 30.5E12, at least 40E12, at least 40.5E12, at least 50E12, at least 50.5E12, at least 60E12, at least 60.5E12, at least 70E12, at least 70.5E12, at least 80E12, at least 80.5E12, at least 90E12, at least 95E12, or at least 100E12 vector genomes, wherein E is a short-hand for base 10 for exponentiation, and wherein xEy refers to x multiplied by base 10 to the y power/exponent.

In some embodiments, a unit dose comprise between 2E12 to 6E12 vector genomes. In some embodiments, a unit dose comprises about 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, or 9.5E12 vector genomes. In some embodiments, a unit dose comprises between 1E12 to 1.5E12, between 1.5E12 to 2E12, between 2E12 to 2.5E12, between 2.5E12 to 3.0E12, between 3.0E12 to 3.5E12, between 3.5E12 to 4.0E12, between 4.0E12 to 4.5E12, between 4.5E12 to 5.0E12, between 5.0E12 to 5.5E12, between 5.5E12 to 6.0E12, between 6.0E12 to 6.5E12, between 6.5E12 to 7.0E12, between 7.0E12 to 7.5E12, between 7.5E12 to 8.0E12, between 8.0E12 to 8.5E12, between 8.5E12 to 9.0E12, between 9.0E12 to 9.5E12, or between 9.5E12 to 10E12 vector genomes. In some embodiments, a unit dose comprises at least 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, or 9.5E12 vector genomes. In some embodiments, a unit dose comprise no more than 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, or 9.5E12 vector genomes.

In some embodiments, viral vector of the disclosure is measured using multiplicity of infection (MOI). In some cases, MOI refers to the ratio, or multiple of vector or viral genomes to the cells to which the nucleic acid can be delivered. In some cases, the MOI is 1×10⁶. In some cases, recombinant viruses of the disclosure can be at least 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, 1×10¹⁵, 1×10¹⁶, 1×10¹⁷ and 1×10¹⁸ MOI. In some cases, recombinant viruses of this disclosure can be from 1×10⁸ to 1×10¹⁵ MOI. In some cases, recombinant viruses of the disclosure can be at most 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³ 1×10¹⁴, 1×10¹⁵, 1×10¹⁶, 1×10¹⁷, and 1×10¹⁸ MOI.

In some embodiments, the nucleic acid may be delivered without the use of a virus (i.e. with a non-viral vector), and may be measured as the quantity of nucleic acid. Generally, any suitable amount of nucleic acid may be used with the pharmaceutical compositions and methods of this disclosure. In some cases, nucleic acid is at least 1 pg, 10 pg, 100 pg, 1 pg, 10 pg, 100 pg, 200 pg, 300 pg, 400 pg, 500 pg, 600 pg, 700 pg, 800 pg, 900 pg, 1 µg, 10 µg, 100 µg, 200 µg, 300 µg, 400 µg, 500 µg, 600 µg, 700 µg, 800 µg, 900 µg, 1 ng, 10 ng, 100 ng, 200 ng, 300 ng, 400 ng, 500 ng, 600 ng, 700 ng, 800 ng, 900 ng, 1 mg, 10 mg, 100 mg, 200 mg, 300 mg, 400 mg, 500 mg, 600 mg, 700 mg, 800 mg, 900 mg 1 g, 2 g, 3 g, 4 g, or 5 g. In some cases, nucleic acid may be at most about 1 pg, 10 pg, 100 pg, 1 pg, 10 pg, 100 pg, 200 pg, 300 pg, 400 pg, 500 pg, 600 pg, 700 pg, 800 pg, 900 pg, 1 µg, 10 µg, 100 µg, 200 µg, 300 µg, 400 µg, 500 µg, 600 µg, 700 µg, 800 µg, 900 µg, 1 ng, 10 ng, 100 ng, 200 ng, 300 ng, 400 ng, 500 ng, 600 ng, 700 ng, 800 ng, 900 ng, 1 mg, 10 mg, 100 mg, 200 mg, 300 mg, 400 mg, 500 mg, 600 mg, 700 mg, 800 mg, 900 mg, 1 g, 2 g, 3 g, 4 g, or 5 g.

In some embodiments, a self-complementary vector (sc) can be used. The use of self-complementary AAV vectors may bypass the requirement for viral second-strand DNA synthesis and may lead to greater rate of expression of the transgene protein, as provided by Wu, Hum Gene Ther. 2007, 18(2):171-82, incorporated by reference herein.

In some aspects, several AAV vectors may be generated to allow selection of the most optimal serotype and promoter for use with the aflibercept transgene.

In some cases, the vector can be a targeted vector, especially a targeted rAAV (e.g., AAV2.7m8) that shows higher infectivity of a specific cell, such as retinal cells, or a photoreceptor, a retinal ganglion cell, a Müller cell, a bipolar cell, an amacrine cell, a horizontal cell, or a retinal pigmented epithelium cell. Viral vectors for use in the disclosure can include those that exhibit low toxicity and/or low immunogenicity in a subject and expresses therapeutically effective quantities of the aflibercept protein in a subject, e.g., human patient.

Disclosed herein are pharmaceutical compositions and methods for delivering a nucleic acid encoding aflibercept into a target retinal cell of a subject using the a rAAV comprising a 7m8 variant capsid protein, or rAAV2.7m8, and a nucleic acid sequence that encodes aflibercept in a non-human primate or a human subject. In some instances, the delivery of aflibercept via gene therapy can be used to at least partially ameliorate or prevent an ocular disease or condition disclosed herein.

In some embodiments, the increase in retinal cell infectivity of rAAV variant (e.g., the 7m8 variant) is at least 5%, at least 10%, at least 20%, at least 30%, at least 40%, at least 50%, at least 60%, at least 70%, at least 80%, at least 90%, or at least 100% as compared to an AAV virion comprising the corresponding parental or unmodified AAV capsid protein. In some embodiments, the increase in infectivity of retinal cells is an increase of between 5% to 100%, between 5% to 95%, between 5% to 90%, between 5% to 85%, between 5% to 80%, between 5% to 75%, between 5% to 70%, between 5% to 65%, between 5% to 60%, between 5% to 55%, between 5% to 50%, between 5% to 45%, between 5% to 40%, between 5% to 35%, between 5% to 30%, between 5% to 25%, between 5% to 20%, between 5% to 15%, between 5% to 10% as compared to an AAV virion comprising the corresponding parental or unmodified AAV capsid protein.

In some embodiments, the increase in retinal cell infectivity of a rAAV variant is at least 1-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, or at least 2-fold compared to an AAV virion comprising the corresponding parental or unmodified AAV capsid protein. In some embodiments, the increase in infectivity is at least 2-fold, at least 3-fold, at least 4-fold, at least 5-fold, at least 6-fold, at least 7-fold, at least 8-fold, at least 9-fold, or at least 10-fold as compared to an AAV virion comprising the corresponding parental AAV capsid protein. In some embodiments, the increase in infectivity is at least 15-fold, at least 20-fold, at least 25-fold, at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, or at least 100-fold compared to an AAV virion comprising the corresponding parental or unmodified AAV capsid protein.

In some embodiments, the increase in retinal cell infectivity is between 10-fold to 100-fold, between 10-fold to 95-fold, between 10-fold to 90-fold, between 10-fold to 85-fold, between 10-fold to 80-fold, between 10-fold to 75-fold, between 10-fold to 70-fold, between 10-fold to 65-fold, between 10-fold to 60-fold, between 10-fold to 55-fold, between 10-fold to 50-fold, between 10-fold to 45-fold, between 10-fold to 40-fold, between 10-fold to 35-fold, between 10-fold to 30-fold, between 10-fold to 25-fold, between 10-fold to 20-fold, or between 10-fold to 15-fold as compared to an AAV virion comprising the corresponding parental or unmodified AAV capsid protein.

In some embodiments, the increase in retinal cell infectivity is between 2-fold to 20-fold, between 2-fold to 19-fold, between 2-fold to 18-fold, between 2-fold to 17-fold, between 2-fold to 16-fold, between 2-fold to 15-fold, between 2-fold to 14-fold, between 2-fold to 13-fold, between 2-fold to 12-fold, between 2-fold to 11-fold, between 2-fold to 10-fold, between 2-fold to 9-fold, between 2-fold to 8-fold, between 2-fold to 7-fold, between 2-fold to 6-fold, between 2-fold to 5-fold, between 2-fold to 4-fold, or between 2-fold to 3-fold as compared to an AAV virion comprising the corresponding parental or unmodified AAV capsid protein.

In some embodiments, an amino acid modification of a capsid protein described herein can confer an increase in an ability to cross an internal limiting membrane (ILM) in an eye of a primate or human subject as compared to the ability of an AAV virion comprising the corresponding parental or unmodified AAV capsid protein to cross the ILM in the eye of the subject. In some embodiments, the increase in the ability to cross the ILM is an increase of at least 5%, at least 10%, at least 20%, at least 30%, at least 40%, at least 50%, at least 60%, at least 70%, at least 80%, at least 90%, or at least 100% as compared to an AAV virion comprising the corresponding parental or unmodified AAV capsid protein. In some embodiments, the increase in the ability to cross the ILM is an increase of between 5% to 100%, between 5% to 95%, between 5% to 90%, between 5% to 85%, between 5% to 80%, between 5% to 75%, between 5% to 70%, between 5% to 65%, between 5% to 60%, between 5% to 55%, between 5% to 50%, between 5% to 45%, between 5% to 40%, between 5% to 35%, between 5% to 30%, between 5% to 25%, between 5% to 20%, between 5% to 15%, or between 5% to 10% as compared to the parental or unmodified AAV capsid protein.

In some embodiments, the increase in the ability to cross the ILM is at least 1-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, or at least 2-fold compared to an AAV virion comprising the corresponding parental AAV capsid protein. In some embodiments, the increase in the ability to cross the ILM is at least 2-fold, at least 3-fold, at least 4-fold, at least 5-fold, at least 6-fold, at least 7-fold, at least 8-fold, at least 9-fold, or at least 10-fold as compared to an AAV virion comprising the corresponding parental AAV capsid protein. In some embodiments, the increase in the ability to cross the ILM is at least 15-fold, at least 20-fold, at least 25-fold, at least 30-fold, at least 35-fold, at least 40-fold, at least 45-fold, at least 50-fold, at least 55-fold, at least 60-fold, at least 65-fold, at least 70-fold, at least 75-fold, at least 80-fold, at least 85-fold, at least 90-fold, or at least 100-fold compared to an AAV virion comprising the corresponding parental or unmodified AAV capsid protein.

In some embodiments, the increase in the ability to cross the ILM is between 10-fold to 100-fold, between 10-fold to 95-fold, between 10-fold to 90-fold, between 10-fold to 85-fold, between 10-fold to 80-fold, between 10-fold to 75-fold, between 10-fold to 70-fold, between 10-fold to 65-fold, between 10-fold to 60-fold, between 10-fold to 55-fold, between 10-fold to 50-fold, between 10-fold to 45-fold, between 10-fold to 40-fold, between 10-fold to 35-fold, between 10-fold to 30-fold, between 10-fold to 25-fold, between 10-fold to 20-fold, or between 10-fold to 15-fold as compared to an AAV virion comprising the corresponding parental or unmodified AAV capsid protein.

In some embodiments, the increase in the ability to cross the ILM is between 2-fold to 20-fold, between 2-fold to 19-fold, between 2-fold to 18-fold, between 2-fold to 17-fold, between 2-fold to 16-fold, between 2-fold to 15-fold, between 2-fold to 14-fold, between 2-fold to 13-fold, between 2-fold to 12-fold, between 2-fold to 11-fold, between 2-fold to 10-fold, between 2-fold to 9-fold, between 2-fold to 8-fold, between 2-fold to 7-fold, between 2-fold to 6-fold, between 2-fold to 5-fold, between 2-fold to 4-fold, or between 2-fold to 3-fold as compared to an AAV virion comprising the corresponding parental or unmodified AAV capsid protein.

### Aflibercept

In some embodiments, a gene therapy is used to deliver a therapeutic transgene comprising a nucleic acid sequence that encodes or expresses aflibercept when administered to an eye or vitreous of an eye of a non-human primate or a human subject. In some embodiments, rAAV comprising a capsid variant (e.g., AAV2.7m8) described herein comprises a heterologous nucleic acid sequence that encodes aflibercept is used to deliver the sequence of the aflibercept gene into retinal cells upon intravitreal or subretinal injection to a subject. In some embodiments, the rAAV comprising the aflibercept gene is formulated for gene therapy and intravitreal injection. In some embodiments, the aflibercept gene refers to a functional fragment or a variant thereof. In some embodiments, the nucleic acid sequence of aflibercept is derived from its amino acid sequence. In some embodiments, the nucleic acid sequence of aflibercept is codon optimized to improve its expression in a subject.

Codon optimization can be achieved with any method known in the art. Codon optimization refers to a process of modifying a nucleic acid sequence for enhanced expression of a gene in target or host cells of interest, e.g., human retinal cells, by replacing at least one codon (e.g., about or more than 1, 2, 3, 4, 5, 10, 15, 20, 25, 50, 100 or more codons) of a native sequence with codons that are used more frequently or are most frequently used in the host cell while maintaining the native amino acid sequence. Codon usage tables are readily available, including for examples, GenScript Codon Usage Frequence Table Tool at http://www.genscript.com/tools/codon-frequency-table; Codon Usage Database at http://www.kazusa.or.jp/codon/; and Nakamura, Y., et al. "Codon usage tabulated from the international DNA sequence databases: status for the year 2000" Nucl. Acids Res. 28:292 (2000).

Aflibercept is a 115 kDa fusion protein, which can be glycosylated. Aflibercept comprises an IgG backbone fused to extracellular VEGF receptor sequences of the human VEGFR-1 and VEGFR-2, and functions like a soluble decoy receptor by binding VEGF-A with a greater affinity than its natural or endogenous receptors. See, for example, Stewart MW. Aflibercept (VEGF Trap-eye): the newest anti-VEGF drug. Br. J. Ophthalmol. 2012 Sep;96(9):1157-8. Aflibercept's high affinity for VEGF interferes or disrupts subsequent binding and activation of native or endogenous VEGF receptors. Reduced VEGF activity can lead to decreased angiogenesis and vascular permeability. Inhibition of placental growth factor PIGF and VEGF-B by aflibercept may also contribute to the treatment of angiogenic conditions. PIGF has been associated with angiogenesis and can be elevated in multiple conditions, such as wet AMD. VEGF-B overexpression can be associated with breakdown of the blood-retinal barrier and retinal angiogenesis. Thus, inhibition of VEGF-A, VEGF-B, and PIGF may all contribute to the efficacy of aflibercept.

The gene product disclosed herein consists of aflibercept, a functional fragment, or a mutant or variant thereof. The amino acid sequence of aflibercept is known in the art: C₄₃₁₈H₆₇₈₈N₁₁₆₄O₁₃₀₄S₃₂, FDA Unique Ingredient Identifier (UNII) is 15C2VL427D. The amino acid sequence of aflibercept is available at DrugBank, accession number DB08885:

SDTGRPFVEMYSEIPEIIHMTEGRELVIPCRVTSPNITVTLKKFPLDTLIPDGKRIIWDSRKG
FIISNATYKEIGLLTCEATVNGHLYKTNYLTHRQTNTIIDVVLSPSHGIELSVGEKLVLNCT
ARTELNVGIDFNWEYPSSKHQHKKLVNRDLKTQSGSEMKKFLSTLTIDGVTRSDQGLYT
CAASSGLMTKKNSTFVRVHEKDKTHTCPPCPAPELLGGPSVFLFPPKPKDTLMISRTPEV
TCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHEALHNH
YTQKSLSLSPG (SEQ ID NO: 1).

The nucleic acid sequence of aflibercept (SEQ ID NO: 2) is illustrated at FIG. 5. In some embodiments, the nucleic acid sequence of aflibercept is codon-optimized for expression in a primate or a human subject. Construction of a synthetic gene corresponding to the aflibercept amino acid sequence has been described in literature, e.g., Kanda A, Noda K, Saito W, Ishida S. Aflibercept Traps Galectin-1, an Angiogenic Factor Associated with Diabetic Retinopathy. Scientific Reports 5:17946 (2015) (describing "VEGF-Trap_{R1R2} (corresponding to aflibercept) cDNA was generated as a synthetic gene by IDT (Coralville, IA)"). Given the available amino acid sequence of aflibercept, any method known in the art can be used to generate the cDNA of aflibercept for use in a gene therapy or a rAAV described herein.

As used herein, "aflibercept" refers to a polypeptide or protein sequence, or a functional fragment or variant or mutant thereof, with at least 75%, 80%, 81%, 82%, 83%, 84%, 85%, 86%, 87%, 88%, 89%, 90%, 91%, 92%, 93%, 94%, 95%, 96%, 97%, 98%, 99% or more, or 100% homology to the aflibercept amino acid sequence identified above . Homology refers to the % conservation of residues of an alignment between two sequences, including, but not limited to functional fragments, sequences comprising insertions, deletions, substitutions, pseudofragments, pseudogenes, splice variants or artificially optimized sequences.

In some cases, the amino acid sequence of aflibercept is at least 75%, 80%, 81%, 82%, 83%, 84%, 85%, 86%, 87%, 88%, 89%, 90%, 91%, 92%, 93%, 94%, 95%, 96%, 97%, 98%, 99%, 99.9%, or 100% homologous to the aflibercept amino acid sequence of SEQ ID NO: 1. In some embodiments, the nucleic acid sequence used in a gene therapy or rAAV disclosed herein is compared to the corresponding cDNA sequence of the aflibercept amino acid sequence identified above, and shows at least 75%, 80%, 81%, 82%, 83%, 84%, 85%, 86%, 87%, 88%, 89%, 90%, 91%, 92%, 93%, 94%, 95%, 96%, 97%, 98%, 99%, 99.9%, or 100% sequence homology between the nucleic acid sequences of aflibercept (e.g., SEQ ID NO: 2). In some cases, aflibercept is at least 80%, 85%, 90%, 91%, 92%, 93%, 94%, 95%, 96%, 97%, 98%, 99%, 99.9%, or 100% spatially homologous to aflibercept (e.g., in terms of its secondary, tertiary, and quaternary structure or conformation). In some cases, aflibercept of the pharmaceutical compositions and methods disclosed herein is at most 80%, 85%, 90%, 91%, 92%, 93%, 94%, 95%, 96%, 97%, 98%, 99%, 99.9%, or 100% spatially homologous to the aflibercept used in the standard of care (e.g., secondary, tertiary, and quaternary structure or conformation).

In some instances, the aflibercept gene product, or aflibercept transgene, as included in a gene therapy based on a rAAV comprises a capsid variant as disclosed herein (e.g., the 7m8 variant), encodes a protein, fusion protein, or polypeptide that has at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or at least 100% homology to the above amino acid sequence of SEQ ID NO: 1, or between the corresponding cDNA sequences of aflibercept (e.g., cDNA of aflibercept sequence used in a gene therapy compared to SEQ ID NO: 2). In some embodiments, methods and pharmaceutical compositions disclosed herein comprise a functional fragment of aflibercept, or a variant or mutant thereof. In some embodiments, the nucleic acid sequence of aflibercept is modified or codon-optimized to enhance its activity, expression, stability, and/or solubility in vivo.

In some embodiments, AAV2.7m8 is used as a gene therapy or delivery system for aflibercept. AAV2.7m8-aflibercept refers to a rAAV2 comprising the 7m8 insertion between positions 587 and 588 in capsid protein VP1 of rAAV2 and a nucleic acid sequence encoding aflibercept.

### Pharmaceutical Compositions

In some embodiments, a pharmaceutical composition is a formulation containing one or more active ingredients, e.g., AAV2.7m8 comprising a nucleic acid sequence that encodes the aflibercept fusion protein, or a fragment or variant thereof, as well as one or more excipients, carriers, stabilizers, or bulking agents, which are suitable for administration to a human patient via intravitreal or subretinal injection to achieve a desired therapeutic or prophylactic effect.

In some embodiments, the pharmaceutical compositions comprising rAAV or AAV2.7m8, and a nucleic acid sequence that encodes the aflibercept fusion protein, or a fragment or variant thereof, are supplied as a reconstituted homogenous solution. In some embodiments, the solution can be a suspension. In some embodiments, the solution is isotonic. In other embodiments, the pharmaceutical compositions comprising rAAV or AAV2.7m8 and a nucleic acid sequence that encodes the aflibercept fusion protein, or a fragment or variant thereof, are supplied in a lyophilized form, and is reconstituted before administration to a patient. In some embodiments, method of treatment or prevention of an eye disease or condition as disclosed herein comprises first reconstituting, dissolving, or solubilizing a lyophilized pharmaceutical composition comprising rAAV (e.g., AAV2.7m8) and a nucleic acid sequence that encodes the aflibercept fusion protein, or a functional fragment or variant thereof in a buffer. In some embodiments, such lyophilized pharmaceutical composition can further comprise a cryoprotectant, surfactant, salt, a stabilizer, or any combination thereof. In some embodiments, a homogenous solution containing the pharmaceutical composition is supplied as a pre-filled syringe.

In some embodiments, pharmaceutical compositions disclosed herein are supplied as a suspension. In some embodiments, a suspension is a solution. In some embodiments, the suspension is refrigerated. In some embodiments, method of treatment or prevention of an eye disease or condition as disclosed herein comprises warming the refrigerated suspension to room temperature and/or agitating the suspension to ensure even distribution before administering or intravitreal injection to a patient. In some embodiments, the suspension is diluted before administering to a patient. In some embodiments, such pharmaceutical composition comprises a surfactant, salt, a stabilizer, or any combination thereof. In some embodiments, a suspension containing the pharmaceutical composition is supplied as a pre-filled syringe.

In some embodiments, the gene therapy or pharmaceutical compositions described herein is provided as a suspension or refrigerated suspension. In some embodiments, the suspension comprises a pharmaceutically acceptable excipient, e.g., surfactant, glycerol, non-ionic surfactant, buffer, glycol, salt, and any combination thereof. In some embodiments, hydrochloric acid and sodium hydroxide are used to adjust the pH of the solution. In some embodiments, the refrigerated suspension is at a neutral pH, or at a pH between 6.5 to 7.5. In some embodiments, the pH of the refrigerated suspension is slightly basic (e.g., pH about 7.5, 8, 8.2, 8.4, 8.5, or 9). In some embodiments, the pH of the suspension or solution is slightly acidic (e.g., pH about 6.5, 6.3, 6.1, 6, 5.5, or 5). In some embodiments, the suspension is a solution. In some embodiments, the suspension comprises micelles. In some embodiments, suspension is agitated and/or warmed to room temperature before administration.

In some embodiments, a gene therapy comprising pharmaceutical composition comprising rAAV (e.g., AAV2.7m8) and the aflibercept nucleic acid sequence is supplied as a kit, comprising lyophilized or freeze-dried pharmaceutical composition (e.g., one unit dose in a vial) disclosed herein and a solution for dissolving, diluting, and/or reconstituting the lyophilized pharmaceutical composition. In some embodiments, the solution for reconstituting or dilution can be supplied as a pre-filled syringe. In some embodiments, a kit comprises a freeze-dried or lyophilized pharmaceutical composition comprising rAAV (e.g., AAV2.7m8) and a solution for reconstituting the pharmaceutical composition to a desired concentration or volume. In some embodiments, the kit includes a buffer that helps to prevent aggregation upon reconstituting the pharmaceutical composition disclosed herein. In some embodiments, the pharmaceutical composition is provided in a pre-filled syringe. In some embodiments, a kit comprises a dual-chamber syringe or container wherein one of the chambers contains a buffer for dissolving or diluting the pharmaceutical composition. In some embodiments, the kit comprises a syringe for injection. In some embodiments, the reconstituted solution is filtered before administration. In some embodiments, the kit comprises a filter or a filter syringe for filtering the reconstituted pharmaceutical composition before administration to a patient.

In some embodiments, for storage stability and convenience of handling, a pharmaceutical composition, comprising rAAV (e.g., AAV2.7m8) and a nucleic acid sequence that encodes the aflibercept fusion protein, can be formulated as a lyophilized, freeze dried, or vacuum dried powder that can be reconstituted with saline, buffer, or water prior to administration to a subject. Alternately, the pharmaceutical composition can be formulated as an aqueous solution, such as a suspension or a homogeneous solution. A pharmaceutical composition can contain rAAV virions or particles comprising a nucleic acid sequence that encodes aflibercept. In some embodiments, a different virus or delivery system, e.g., nanoparticles or lipid-based complexes, can be used to deliver the nucleic acid sequence that encodes aflibercept. Various excipients, such as phosphate, PBS, or Tris buffer, glycol, glycerol, saline, surfactant (e.g., pluronic or polysorbate), or any combination thereof, can be used to stabilize a pharmaceutical composition. Additionally, cryoprotectants, such as alcohols can be used as a stabilizer under freezing or drying conditions of lyophilization. In some embodiments, the gene therapy is provided as a suspension or a refrigerated suspension.

In some embodiments, a suspension or a reconstituted form of the lyophilized pharmaceutical composition comprising the aflibercept gene therapy as disclosed herein has a volume of about 10, 20, 30, 40, 50, 60, 70, 80, 90, 100, 200, 300, 400, 500, 600, 700, 800, 900, or 1000 µL. In some embodiments, the suspension of the pharmaceutical composition comprising the aflibercept gene therapy as disclosed herein has a volume of between 0.1 to 0.5 mL, between 0.1 to 0.2 mL, between 0.3 to 0.5 mL, between 0.5-1.0 mL, between 0.5-0.7 mL, between 0.6 to 0.8 mL, between 0.8 to 1 mL, between 0.9 to 1.1 mL, between 1.0 to 1.2 mL, or between 1.0 to 1.5 mL. In other embodiments, the volume is no more than 0.1, 0.2, 0.3, 0.4, 0.5, 0.6, 0.7, 0.8, 0.9, 1.0, 1.1, 1.2, 1.3, 1.4, or 1.5 mL.

In some embodiments, pharmaceutical compositions disclosed herein are designed, engineered, or adapted for administration to a primate (e.g., non-human primate and human subjects) via intravitreal or subretinal injection. In some embodiments, a pharmaceutical composition comprising rAAV virions comprising a nucleic acid sequence that encodes aflibercept is formulated for intravitreal injection into an eye of a subject. In some embodiments, the pharmaceutical composition is formulated to or reconstituted to a concentration that allows intravitreal injection of a volume not more than about or not more than 2, 2.5, 5, 10, 15, 20, 25, 30, 35, 40, 45, 50, 55, 60, 65, 70, 75, 80, 85, 90, 95, 100, 110, 120, 130, 140, 150, 160, 170, 180, 190, or 200 µL. In some embodiments, a unit dose of the pharmaceutical composition comprises a volume not more than about or not more than 2, 2.5, 5, 10, 15, 20, 25, 30, 35, 40, 45, 50, 55, 60, 65, 70, 75, 80, 85, 90, 95, 100, 110, 120, 130, 140, 150, 160, 170, 180, 190, or 200 µL. In some embodiments, methods of treatment disclosed herein comprises intravitreal injection of a volume of about 2, 5, 10, 15, 20, 25, 30, 35, 40, 45, 50, 55, 60, 65, 70, 75, 80, 85, 90, 95, 100, 110, 120, 130, 140, 150 µL of a solution comprising a rAAV (e.g., AAV2.7m8) and a nucleic acid sequence that encodes aflibercept.

In some instances, an AAV2.7m8 virion comprising a nucleic acid sequence of the aflibercept transgene described herein can be a component of a gene therapy pharmaceutical composition. In some embodiments, a rAAV virion of any serotype comprising the 7m8 variant capsid protein as described herein can be used to make a freeze-dried or lyophilized pharmaceutical composition or a suspension thereof. In some embodiments, the gene therapy is formulated as a refrigerated suspension. In some embodiments, the rAAV virion is rAAV2. In some embodiments, the lyophilized or suspension of the pharmaceutical composition comprises rAAV2 having the 7m8 variant capsid protein and a DNA sequence that encodes aflibercept, a functional fragment, or variant thereof. In some embodiments, the suspension is refrigerated.

In some embodiments, a pharmaceutical composition disclosed herein is adapted for gene therapy or for intravitreal delivery of aflibercept as the therapeutic agent in human patients or non-human primates. In some embodiments, a unit dose of the pharmaceutical composition comprises between 1×10¹⁰ to 1×10¹³ viral genomes (vg). In some embodiments, a unit dose comprises about 2.1×10¹¹, about 2.1×10¹², or about 2.1×10¹³ vector genome. In some embodiments, a unit dose of the pharmaceutical composition of the disclosure is 1×10¹⁰ to 3×10¹² vector genomes. In some cases, a unit dose of the pharmaceutical composition of the disclosure is 1×10⁹ to 3×10¹³ vector genomes. In some cases, a unit dose of the pharmaceutical composition of the disclosure is 1 ×10¹⁰ to 1×10¹¹ vector genomes. In some cases, a unit dose of the pharmaceutical composition of the disclosure is 1×10⁸ to 3×10¹⁴ vector genomes. In some cases, a unit dose of the pharmaceutical composition of the disclosure is at least 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, 1×10¹⁵, 1×10¹⁶, 1×10¹⁷, or 1×10¹⁸ vector genomes. In some cases, a unit dose of the pharmaceutical composition of the disclosure is 1×10¹⁰ to 5×10¹³ vector genomes. In some cases, a unit dose of the pharmaceutical composition of the disclosure is at most about 1×10⁸, 1×10⁹, 1×10¹⁰, 1×10¹¹, 1×10¹², 1×10¹³, 1×10¹⁴, 1×10¹⁵, 1×10¹⁶, 1×10¹⁷, and 1×10¹⁸ vector genomes.

In some cases, a unit dose of the pharmaceutical composition of the disclosure can be measured as pfu (plaque forming units). In some cases, the pfu of the unit dose of the pharmaceutical composition of the disclosure can be about 1×10⁸ to about 1×10¹² pfu. In some cases, the pfu of the unit dose of the pharmaceutical composition of the disclosure can be at least about 1×10⁸, 2×10⁸, 3×10⁸, 4×10⁸, 5×10⁸, 6×10⁸, 7×10⁸, 8×10⁸, 9×10⁸, 1×10⁹, 2×10⁹, 3×10⁹, 4×10⁹, 5×10⁹, 6×10⁹, 7×10⁹, 8×10⁹, 9×10⁹, 1×10¹⁰, 2×10¹⁰, 3×10¹⁰, 4×10¹⁰, 5×10¹⁰, 6×10¹⁰, 7×10¹⁰, 8×10¹⁰, 9×10¹⁰, 1×10¹¹, 2×10¹¹, 3×10¹¹, 4×10¹¹, 5×10¹¹, 6×10¹¹, 7×10¹¹, 8×10¹¹, 9×10¹¹ or 1×10¹² pfu. In some cases, the pfu of the unit dose of the pharmaceutical composition of the disclosure can be at most about 1×10⁸, 2×10⁸, 3×10⁸, 4×10⁸, 5×10⁸, 6×10⁸, 7×10⁸, 8×10⁸, 9×10⁸, 1×10⁹, 2×10⁹, 3×10⁹, 4×10⁹, 5×10⁹, 6×10⁹, 7×10⁹, 8×10⁹, 9×10⁹, 1×10¹⁰, 2×10¹⁰, 3×10¹⁰, 4×10¹⁰, 5×10¹⁰, 6×10¹⁰, 7×10¹⁰, 8×10¹⁰, 9×10¹⁰, 1×10¹¹, 2×10¹¹, 3×10¹¹, 4×10¹¹, 5×10¹¹, 6×10¹¹, 7×10¹¹, 8×10¹¹, 9×10¹¹ or 1×10¹² pfu.

In some cases, the viral vector of the disclosure may be measured as vector genomes (vg). In some cases, a unit dose of the pharmaceutical composition of the disclosure can be 1×10¹⁰ to 1×10¹³ vector genomes. In some cases, a unit dose of the pharmaceutical composition of the disclosure can be 1×10⁹ to 1×10¹⁴ vector genomes. In some cases, a unit dose of the pharmaceutical composition of the disclosure can be 1×10¹⁰ to 1×10¹¹ vector genomes. In some cases, a unit dose of the pharmaceutical composition of the disclosure can be 1×10⁸ to 1×10¹⁵ vector genomes. In some cases, a unit dose of the pharmaceutical composition of the disclosure is at least 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, 1×10¹⁵, 1×10¹⁶, 1×10¹⁷ and 1×10¹⁸ vector genomes. In some cases, a unit dose of the pharmaceutical composition of the disclosure is 1×10⁸ to 1×10¹⁵ vector genomes. In some cases, a unit dose of the pharmaceutical composition of the disclosure is at most about 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, 1×10¹⁵, 1×10¹⁶, 1×10¹⁷ and 1×10¹⁸ vector genomes. In some embodiments, a unit dose is between 10¹⁰ to 10¹¹ , between 10¹¹ to 10¹², between 10¹⁰ to 10¹², between 10¹² to 10¹³, between 10¹¹ to 10¹³, between 10¹² to 10¹³, between 10¹² to 10¹⁴, between 10¹¹ to 10¹⁴, between 10¹¹ to 10¹⁵, between 10¹² to 10¹⁵, between 10¹³ to 10¹⁴, between 10¹⁴ to 10¹⁵, between 10¹⁵ to 10¹⁶, between 10¹⁶ to 10¹⁷, between 10¹⁷ to 10¹⁸, between 10¹⁸ to 10¹⁹, or between 10¹⁹ to 10²⁰ vector genomes.

In some embodiments, a unit dose of the pharmaceutical composition of the disclosure is between 1×10¹⁰ to 2×10¹⁰, between 2×10¹⁰ to 3×10¹⁰, between 3×10¹⁰ to 4×10¹⁰, between 4×10¹⁰ to 5×10¹⁰, between 5×10¹⁰ to 6×10¹⁰, between 6×10¹⁰ to 7×10¹⁰, between 7×10¹⁰ to 8×10¹⁰, between 8×10¹⁰ to 9×10¹⁰, between 9×10¹⁰ to 10×10¹⁰, between 1×10¹¹ to 2×10¹¹, between 2×10¹¹ to 3×10¹¹, between 2×10¹¹ to 2.5×10¹¹, between 2.5×10¹¹ to 3×10¹¹, between 3×10¹¹ to 4×10¹¹, between 4×10¹¹ to 5×10¹¹, between 5×10¹¹ to 6×10¹¹, between 6×10¹¹ to 7×10¹¹, between 7×10¹¹ to 8×10¹¹, between 8×10¹¹ to 9×10¹¹, between 9×10¹¹ to 10×10¹¹, between 1×10¹² to 2×10¹², between 2×10¹² to 3×10¹², between 2.5×10¹² to 3×10¹², between 3×10¹² to 4×10¹², between 4×10¹² to 5×10¹², between 5×10¹² to 6×10¹², between 6×10¹² to 7×10¹², between 7×10¹² to 8×10¹², between 8×10¹² to 9×10¹², between 9×10¹² to 10×10¹², between 1×10¹³ to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 9×10¹³ to 10×10¹³ vector genomes.

In some embodiments, a unit dose of rAAV of this disclosure is between 2.1×10¹¹ or between 2.1×10¹² vector genomes. In some embodiments, a unit dose of rAAV of this disclosure is between 10¹⁰ to 10¹³, between 10¹⁰ to 10¹¹, between 10¹¹ to 10¹², between 10¹² to 10¹³, or between 10¹³ to 10¹⁴ vector genomes.

In some embodiments, a unit dose of rAAV of this disclosure is between 1×10¹⁰ to 2×10¹⁰, between 2×10¹⁰ to 4×10¹⁰, between 3×10¹⁰ to 5×10¹⁰, between 4×10¹⁰ to 6×10¹⁰, between 5×10¹⁰ to 7×10¹⁰, between 6×10¹⁰ to 8×10¹⁰, between 7×10¹⁰ to 9×10¹⁰, between 8×10¹⁰ to 10¹¹, between 1×10¹¹ to 2×10¹¹, between 2×10¹¹ to 4×10¹¹, between 3×10¹¹ to 5×10¹¹, between 4×10¹¹ to 6×10¹¹, between 5×10¹¹ to 7×10¹¹, between 6×10¹¹ to 8×10¹¹, between 7×10¹¹ to 9×10¹¹, between 8×10¹¹ to 10×10¹¹, between 1×10¹² to 3×10¹², between 2×10¹² to 4×10¹², between 3×10¹² to 5×10¹², between 4×10¹² to 6×10¹², between 5×10¹² to 7×10¹², between 6×10¹² to 8×10¹², between 7×10¹² to 9×10¹², between 8×10¹² to 10×10¹², between 1×10¹³ to 5×10¹³, between 5×10¹³ to 10×10¹³, between 10¹² to 5×10¹², between 5×10¹² to 1×10¹³, between 7×10¹² to 1×10¹³, between 8×10¹² to 2×10¹³, between 9×10¹² to 2×10¹³, between 9×10¹² to 2×10¹³, between 9×10¹² to 4×10¹³, between 1×10¹³ to 3×10¹³, between 1×10¹³ to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 8×10¹³ to 1×10¹⁴ vector genomes.

In some embodiments, a lower concentration (e.g., vector genomes) is used for a unit dose to prevent aggregation, which can occur at higher concentrations. In some embodiments, a higher concentration, e.g., higher vector genomes, is selected for a unit dose to increase efficacy of the gene therapy, or to maximize the delivery of the aflibercept transgene in one injection or in a one-time administration of the gene therapy. In some embodiments, higher concentrations of the pharmaceutical compositions disclosed herein allow smaller volumes of injection, which can reduce adverse effects associated with intravitreal injection, e.g., elevated intraocular pressure, inflammation, irritation, or pain.

In some cases, a unit dose of the pharmaceutical composition of the disclosure can be measured using multiplicity of infection (MOI). In some cases, MOI can refer to the ratio, or multiple of vector or viral genomes to the cells to which the nucleic may be delivered. In some cases, the MOI can be 1×10⁶. In some cases, the MOI can be between about 1×10⁵ to about 1×10⁷. In some cases, the MOI may be 1×10⁴-1×10⁸. In some cases, recombinant viruses of the disclosure can be at least about 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, 1×10¹⁵, 1×10¹⁶, 1×10¹⁷ and 1×10¹⁸ MOI. In some cases, recombinant viruses of this disclosure can be from about 1×10⁸ to about 1×10¹⁵ MOI. In some cases, recombinant viruses of the disclosure can be at most about 1×10¹, 1×10², 1×10³, 1×10⁴, 1×10⁵, 1×10⁶, 1×10⁷, 1×10⁸, 1×10⁹, 1×10¹⁰, 1×10¹¹, 1×10¹², 1×10¹³, 1×10¹⁴, 1×10¹⁵, 1×10¹⁶, 1×10¹⁷ and 1×10¹⁸ MOI. In some embodiments, the MOI is between 1×10¹⁰ to 2×10¹⁰, between 2×10¹⁰ to 4×10¹⁰, between 3×10¹⁰ to 5×10¹⁰, between 4×10¹⁰ to 6×10¹⁰, between 5×10¹⁰ to 7×10¹⁰, between 6×10¹⁰ to 8×10¹⁰, between 7×10¹⁰ to 9×10¹⁰, between 8×10¹⁰ to 10¹¹, between 1×10¹¹ to 2×10¹¹, between 2×10¹¹ to 4×10¹¹, between 3×10¹¹ to 5×10¹¹, between 4×10¹¹ to 6×10¹¹, between 5×10¹¹ to 7×10¹¹, between 6×10¹¹ to 8×10¹¹, between 7×10¹¹ to 9×10¹¹, between 8×10¹¹ to 10×10¹¹, between 1×10¹² to 3×10¹², between 2×10¹² to 4×10¹², between 3×10¹² to 5×10¹², between 4×10¹² to 6×10¹², between 5×10¹² to 7×10¹², between 6×10¹² to 8×10¹², between 7×10¹² to 9×10¹², between 8×10¹² to 10×10¹², between 1×10¹³ to 5×10¹³, between 5×10¹³ to 10×10¹³, between 10¹² to 5×10¹², between 5×10¹² to 1×10¹³, between 7×10¹² to 1×10¹³, between 8×10¹² to 2×10¹³, between 9×10¹² to 2×10¹³, between 9×10¹² to 2×10¹³, between 9×10¹² to 4×10¹³, between 1×10¹³ to 3×10¹³, between 1×10¹³ to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 8×10¹³ to 1×10¹⁴.

Pharmaceutical compositions suitable for ocular use include sterile aqueous solutions or dispersions and sterile powders for the extemporaneous preparation of sterile injectable solutions, suspension, or dispersion. For intravitreal administration, suitable carriers include physiological saline, bacteriostatic water, phosphate buffered saline (PBS), and/or an isotonic agent, e.g., glycerol. In all cases, the pharmaceutical composition must be sterile and should be fluid to the extent that easy syringability or injectability exists. It must be stable under the conditions of manufacture and storage and must be preserved against the contaminating action of microorganisms such as bacteria and fungi. In some embodiments, the pharmaceutical composition can include an isotonic agent, such as a salt or glycerol. In some embodiments, a surfactant or a stabilizer is added to the pharmaceutical composition to prevent aggregation.

In some instances, the excipient can be a carrier. A carrier can be a solvent or dispersion medium containing, for example, water, saline, ethanol, a polyol (for example, glycerol, propylene glycol, and liquid polyethylene glycol, and the like), and any combination thereof. The proper fluidity can be maintained, for example, by the use of a coating such as lecithin, by the maintenance of the required particle size in the case of dispersion and by the use of surfactants such as polysorbates (e.g., Tween^{™}, polysorbate 20, polysorbate 80), sodium dodecyl sulfate (sodium lauryl sulfate), lauryl dimethyl amine oxide, cetyltrimethylammonium bromide (CTAB), polyethoxylated alcohols, polyoxyethylene sorbitan, octoxynol (Triton X100^{™}), N,N-dimethyldodecylamine-N-oxide, hexadecyltrimethylammonium bromide (HTAB), polyoxyl 10 lauryl ether, Brij 721^{™}, bile salts (sodium deoxycholate, sodium cholate), pluronic acids (F-68, F-127), polyoxyl castor oil (Cremophor^{™}) nonylphenol ethoxylate (Tergitol^{™}), cyclodextrins and, ethylbenzethonium chloride (Hyamine^{™}) Prevention of the action of microorganisms can be achieved by various antibacterial and antifungal agents, for example, parabens, chlorobutanol, phenol, ascorbic acid, cresol, thimerosal, and the like. In many cases, it will be preferable to include isotonic agents, for example, sugars, polyalcohols such as mannitol, sorbitol, sodium chloride in the composition. Prolonged absorption of the internal compositions can be brought about by including in the composition an agent which delays absorption, for example, aluminum monostearate and gelatin. In some embodiments, the pharmaceutical carrier includes sodium phosphate, sodium chloride, polysorbate, and sucrose. In some embodiments, a pharmaceutical composition comprises a surfactant, e.g., non-ionic surfactant such as polysorbate, poloxamer, or pluronic. In some embodiments, the addition of a non-ionic surfactant reduces aggregation in the pharmaceutical composition.

In some embodiments, pharmaceutical compositions useful for the present disclosure can be packaged in a kit to facilitate application of the present disclosure. In some aspects, the present method provides for a kit comprising a recombinant nucleic (e.g., rAAV or rAAV2.7m8 comprising the nucleic acid sequence of aflibercept) of the disclosure. In some aspects, the present method provides for a kit comprising a lyophilized form of a recombinant virus of the disclosure and a solution for reconstituting the virus before administration to a patient. In some embodiments, a kit comprises: a recombinant virus provided herein, and instructions to administer to an eye or retinal cells of a subject in a therapeutically effective amount of the recombinant virus. In some aspects, the kit comprises pharmaceutically acceptable salts or solutions for administering the recombinant virus. Optionally, the kit can further comprise instructions for suitable operational parameters in the form of a label or a separate insert. For example, the kit may have standard instructions informing a physician or laboratory technician to prepare a unit dose of recombinant virus and/or to reconstitute the lyophilized compositions. In some embodiments, optionally, the kit further comprises a device for administration, such as a syringe, filter needle, extension tubing, cannula, or subretinal injector.

In some embodiments, the pharmaceutical composition is provided as a suspension or refrigerated suspension. In some embodiments, the suspension or refrigerated suspension is provided in a kit, which can include a syringe or a buffer for dilution. In some embodiments, the suspension or refrigerated suspension is provided as a pre-filled syringe.

In some embodiments, any suitable method can be used in the biochemical purification of recombinant viruses (e.g., rAAV) for use in a pharmaceutical composition as described herein. Recombinant AAV viruses can be harvested directly from cells, or from the culture media comprising cells. Virus can be purified using various biochemical means, such as gel filtration, filtration, chromatography, affinity purification, gradient ultracentrifugation, or size exclusion methods before lyophilizing or before formulating as a suspension.

### Indications

In some cases, rAAV virion of any serotype comprising the 7m8 variant (e.g., rAAV2.7m8) or a pharmaceutical composition thereof as described herein can at least partially ameliorate an eye condition or disease associated with neovascularization of the eye, or associated with CNV. In some embodiments, a rAAV virion comprising a capsid variant protein is used to deliver aflibercept, a functional fragment, or variant thereof, into an eye of a human subject.

Indications approved for aflibercept fusion protein include neovascular (wet) age-related macular degeneration (AMD), macular edema following retinal vein occlusion (RVO), diabetic macular edema (DME) and diabetic retinopathy (DR) in patients with DME. In some cases, methods and pharmaceutical compositions disclosed herein can be used to prevent or treat an eye condition or disease for which aflibercept is approved or indicated for. In some embodiments, a gene therapy (e.g., AAV2.7m8 based gene therapy) is used to treat or prevent an eye condition or disease that is responsive to aflibercept, including, but not limited to, CNV, wet AMD, dry AMD, DME, RVO, macular edema following RVO, and diabetic retinopathy in patients with DME. In some embodiments, a rAAV gene therapy is used to treat or prevent any eye condition or disorder characterized by neovascularization or CNV. In another aspect, the present disclosure provides pharmaceutical compositions provided herein for the treatment of diseases such as AMD, DME, RVO, angiogenesis related diseases, cancer, autoimmune diseases, infectious disease organisms, and the like.

In some embodiments, the eye condition can be diabetic macular edema. Diabetic macular edema (DME) is a swelling of the retina in diabetes mellitus due to leaking of fluid from blood vessels within the macula. The macula is the central portion of the retina, a small area rich in cones, the specialized nerve endings that detect color and upon which daytime vision depends. As macular edema develops, blurring occurs in the middle or just to the side of the central visual field. Visual loss from diabetic macular edema can progress over a period of months and make it impossible to focus clearly. Common symptoms of DME are blurry vision, floaters, double vision, and eventually blindness if it goes untreated. In some embodiments, methods and pharmaceutical compositions as disclosed herein are used to treat DME.

In some embodiments, the eye condition can be a retinal vein occlusion. Retinal vein occlusion is a blockage of the small veins that carry blood away from the retina. The retina is the layer of tissue at the back of the inner eye that converts light images to nerve signals and sends them to the brain. Retinal vein occlusion is most often caused by hardening of the arteries (atherosclerosis) and the formation of a blood clot. Blockage of smaller veins (branch veins or BRVO) in the retina often occurs in places where retinal arteries that have been thickened or hardened by atherosclerosis cross over and place pressure on a retinal vein. Symptoms of retinal vein occlusion can include a sudden blurring or vision loss in all or part of one eye. In some embodiments, methods and pharmaceutical compositions as disclosed herein are used to treat retinal vein occlusion.

In some embodiments, the eye condition can be choroidal neovascularization (CNV), also known as wet AMD. Choroidal neovascularization can involve the growth of new blood vessels that originate from the choroid through a break in the Bruch membrane into the sub-retinal pigment epithelium (sub-RPE) or subretinal space, which can be a major cause of visual loss. CNV can create a sudden deterioration of central vision, noticeable within a few weeks. Other symptoms can include color disturbances, and metamorphopsia (distortions in which straight lines appears wavy). Hemorrhaging of the new blood vessels can accelerate the onset of symptoms of CNV. CNV may also include feeling of pressure behind the eye. In some embodiments, methods and pharmaceutical compositions as disclosed herein are used to treat CNV or an eye condition associated with neovascularization.

The advanced "wet" form (neovascular or exudative) of AMD is less common, but may frequently cause a rapid and often substantial loss of central vision in patients. In the wet form of AMD, choroidal neovascularization forms and develops into a network of vessels that may grow under and through the retinal pigment epithelium. As this is accompanied by leakage of plasma and/or hemorrhage into the subretinal space, there could be severe sudden loss of central vision if this occurs in the macula. The term "AMD", if not otherwise specified, can be either dry AMD or wet AMD. The present disclosure contemplates treatment or prevention of AMD, wet AMD and/or dry AMD. In some embodiments, methods and pharmaceutical compositions as disclosed herein are used to treat AMD.

In some embodiments, methods and pharmaceutical compositions as disclosed herein are used to prevent or treat an eye disease or condition that is responsive to aflibercept in vivo.

In some embodiments, methods and pharmaceutical compositions disclosed herein, i.e., AAV gene therapy comprising aflibercept, a functional fragment or variant thereof, results in a reduction in neovascularization or CNV, as measured by percentage of grade IV lesions following CNV formation according to color fundus photography, by at least 5%, at least 6%, at least7%, at least 8%, at least 9%, at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% as compared to a vehicle or buffer control.

In some embodiments, methods and pharmaceutical compositions disclosed herein, i.e., AAV gene therapy comprising aflibercept, a functional fragment or variant thereof, results in a reduction in neovascularization or CNV, as measured by percentage of grade IV lesions following CNV formation according to color fundus photography, that is comparable to aflibercept or a non-gene therapy-based aflibercept. In some embodiments, the reduction in CNV, or the therapeutic effect, lasts longer with the administration of a gene therapy comprising aflibercept as compared to administration with a non-gene therapy-based aflibercept or a protein solution of aflibercept. In some embodiments, the therapeutic effect of aflibercept gene therapy lasts for at least 1 year, 1.5, 2, 3, 4, 5, 6, 7, 8, 9, 10 or more years after a single intravitreal injection. In some embodiments, pharmaceutical compositions disclosed herein inhibit or sequester endogenous VEGF and/or PIGF.

### Methods of use

In some embodiments, present disclosure provides a method for treating a pathological angiogenesis related eye disease, comprising administering a pharmaceutically effective amount of the pharmaceutical compositions provided herein to a human subject in need of such treatment. In some embodiments, the disease is selected from the group of ocular neovascular diseases including age-related macular degeneration (AMD), wet-AMD, dry-AMD, retinal neovascularization, choroidal neovascularization diabetic retinopathy, proliferative diabetic retinopathy, retinal vein occlusion, central retinal vein occlusion, branched retinal vein occlusion, diabetic macular edema, diabetic retinal ischemia, ischemic retinopathy and diabetic retinal edema, and any combination thereof.

In some embodiments, pharmaceutical compositions comprising a rAAV comprising a variant capsid protein (e.g., rAAV.7m8) and a nucleic acid sequence that encodes aflibercept is used to treat or prevent AMD, including dry AMD and wet AMD. In some embodiments, pharmaceutical compositions comprising a rAAV comprising a variant capsid protein (e.g., rAAV.7m8) and a nucleic acid sequence that encodes aflibercept is used to treat or prevent CNV, or reduce grade IV CNV lesions. In some embodiments, pharmaceutical compositions comprising a rAAV comprising a variant capsid protein (e.g., rAAV.7m8) and a nucleic acid sequence that encodes aflibercept is used to treat or prevent any one of AMD, wet-AMD, dry-AMD, retinal neovascularization, choroidal neovascularization diabetic retinopathy, proliferative diabetic retinopathy, retinal vein occlusion, central retinal vein occlusion, branched retinal vein occlusion, RVO, diabetic macular edema, diabetic retinal ischemia, ischemic retinopathy and diabetic retinal edema, DR in patients with DME, and any combination thereof.

In some embodiments, the method of treating AMD, DME, RVO, or DR comprises pre-treating a patient with EYLEA^{®} before administering a gene therapy comprising a nucleic acid sequence of aflibercept to the same patient. In some embodiments, a patient is pre-treated with EYLEA^{®} before receiving a one-time dose of the aflibercept gene therapy, as disclosed herein. In some embodiments, a patient is responsive to aflibercept before receiving a one-time dose of the aflibercept gene therapy, as disclosed herein. In some embodiments, a patient who is responsive to aflibercept or who was pre-treated with aflibercept is treated with aflibercept gene therapy, as disclosed herein, followed by a period of at least 1.5, 2, 3, 4, 5, 6, 7, 8, 9, 10 or more years, or more than 1.5, 2, 3, 4, 5, 6, 7, 8, 9, 10 or more years during which the patient does not receive either EYLEA^{®} or aflibercept gene therapy. In some cases, after a patient receives an intravitreal injection of the aflibercept gene therapy, the patient does not begin receiving EYLEA^{®} or another standard of care injection, or another approved therapy (e.g., ranibizumab or bevacizumab injection) until at least 1.5, 2, 3, 4, 5, 6, 7, 8, 9, 10 or more years have lapsed after gene therapy treatment. In some embodiments, a method of treatment comprises assessing or evaluating patients for responsiveness to aflibercept (e.g., by immunoassay or blood test) before administer aflibercept gene therapy to the patient.

In some embodiments, the aflibercept gene therapy disclosed herein is a one-time administration. In some embodiments, after a patient receives a unit dose of the aflibercept gene therapy disclosed herein, the patient does not need to use any other aflibercept-based therapy.

In some cases, patients who experience adverse effects associated with repeated intravitreal injections of EYLEA^{®} or another approved therapy, e.g., inflammation, elevated intraocular pressure, or bacterial infection, can be candidates for treatment with the aflibercept gene therapy disclosed herein. In some cases, such risks are lower in gene therapy because it requires only one injection in a patient's lifetime or is given no more than once in at least 2, 5, 10, 20, 30, 40, 50 or more years. In some cases, treatment with the aflibercept gene therapy disclosed herein can be more cost-effective than protein-based injections because a gene therapy's therapeutic effects can last longer, and the cost of a one-time gene therapy injection may be lower than the combined cost of multiple, repeated injections of a protein.

Also, by not requiring repeated injections, gene therapy addresses the patient compliance and adherence challenge associated with therapies that require repeated injections, as non-compliance (e.g., when a patient forgets or misses one or more scheduled injection) can result in vision loss and deterioration of the eye disease or condition. The rate of non-compliance and non-adherence to treatment regimens that require repeated or frequent trips to medical offices for administration is higher among elderly patients, who are most impacted by AMD. Therefore, delivering aflibercept into an eye of a patient via gene therapy, e.g., as a one-time intravitreal injection, can provide a more convenient treatment option for patients and improve patient outcomes by addressing the non-compliance and non-adherence problem.

In some embodiments, a method of use comprises pre-treating a human patient or subject with a drug that is considered the current standard of care, e.g., aflibercept protein injection, ranibizumab injection, or bevacizumab injection, determining the patient's responsiveness to aflibercept, and administering the aflibercept gene therapy described herein to the patient who is responsive to aflibercept. Determining a patient's responsiveness to aflibercept can include, but not limited to, blood tests, immunoassay, ex vivo experiments, or administration of the aflibercept protein injection to the patient and assaying the patient for responsiveness to aflibercept.

In some embodiments, method of use of the aflibercept gene therapy described herein includes reconstituting a lyophilized form of the pharmaceutical composition described herein (i.e., rAAV2.7m8 comprising the aflibercept nucleic acid sequence) according to the drug label and administering said reconstituted aflibercept gene therapy to a subject or human patient. In some embodiments, the aflibercept gene therapy is provided as a suspension. In some embodiments, the suspension is agitated before administration. In some embodiments, the suspension is refrigerated. In some embodiments, the refrigerated suspension is warmed to room temperature before administration. In some embodiments, such human patient was pre-treated with an aflibercept injection or another protein drug injection, e.g., ranibizumab injection or bevacizumab injection. In some embodiments, such patient receives no more than one injection or administration of the rAAV2.7m8-aflibercept gene therapy for at least 2, 3, 4, 5, 6, 7, 8, 9, 10 or more years; or receives no more than one injection or administration of the rAAV2.7m8-aflibercept gene therapy in more than 2, 3, 4, 5, 6, 7, 8, 9, 10 or more years.

In some embodiments, also disclosed herein are methods of preventing or treating an eye condition or disease, the method comprising administering to an individual in need thereof, e.g., an individual with an eye condition or disease responsive to aflibercept, an effective amount of a rAAV virion comprising a nucleic acid sequence that encodes aflibercept as described herein or a pharmaceutical composition thereof. In some embodiments, rAAV2.7m8-aflibercept virion can be administered via intraocular injection, by intravitreal injection, by subretinal injection, or by any other convenient mode or route of administration into an eye of an individual. Other convenient modes or routes of administration can include, e.g., intravenous, topical, eye drops, etc. In some embodiments, methods and pharmaceutical compositions disclosed herein involve administration by intravitreal injection.

In some embodiments, the gene therapy or pharmaceutical compositions described herein is provided as a refrigerated suspension. In some embodiments, the refrigerated suspension comprises a pharmaceutically acceptable excipient, e.g., surfactant, glycerol, non-ionic surfactant, buffer, glycol, salt, and any combination thereof. In some embodiments, hydrochloric acid and sodium hydroxide are used to adjust the pH of the solution. In some embodiments, the suspension is at a neutral pH, or at a pH between 6.5 to 7.5. In some embodiments, the pH of the suspension is slightly basic (e.g., pH about 7.5, 8, 8.2, 8.4, 8.5, or 9). In some embodiments, the pH of the suspension or solution is slightly acidic (e.g., pH about 6.5, 6.3, 6.1, 6, 5.5, or 5). In some embodiments, the suspension is a solution. In some embodiments, the refrigerated suspension comprises micelles. In some embodiments, refrigerated suspension is agitated before administration. In some embodiments, the refrigerated suspension is stored at temperatures between 35F and 46F (2C and 8C). In some embodiments, the refrigerated suspension is warmed to room temperature before administration to a patient.

A "therapeutically effective amount" as described herein can be a relatively broad range that can be determined through clinical trials. For injection directly into the eye or intravitreal injection, a therapeutically effective dose can be on the order of from 10¹¹ to 10¹² or from 10¹² to 10¹³ vector genomes of 7m8-aflibercept. In some embodiments, a unit dose or a therapeutically effective amount of 7m8-aflibercept is between 10¹⁰ to 10¹¹, between 10¹¹ to 10¹², between 10¹⁰ to 10¹², between 10¹² to 10¹³, between 10¹¹ to 10¹³, between 10¹² to 10¹³, between 10¹² to 10¹⁴, between 10¹¹ to 10¹⁴, between 10¹¹ to 10¹⁵, between 10¹² to 10¹⁵, between 10¹³ to 10¹⁴, between 10¹⁴ to 10¹⁵, between 10¹⁵ to 10¹⁶, between 10¹⁶ to 10¹⁷, between 10¹⁷ to 10¹⁸, between 10¹⁸ to 10¹⁹, or between 10¹⁹ to 10²⁰ vector genomes. In some embodiments, a unit dose of the pharmaceutical composition comprising 7m8-aflibercept of the disclosure is between 1×10¹⁰ to 2×10¹⁰, between 2×10¹⁰ to 3×10¹⁰, between 3×10¹⁰ to 4×10¹⁰, between 4×10¹⁰ to 5×10¹⁰, between 5×10¹⁰ to 6×10¹⁰, between 6×10¹⁰ to 7×10¹⁰, between 7×10¹⁰ to 8×10¹⁰, between 8×10¹⁰ to 9×10¹⁰, between 9×10¹⁰ to 10×10¹⁰, between 1×10¹¹ to 2×10¹¹, between 2×10¹¹ to 3×10¹¹, between 2×10¹¹ to 2.5×10¹¹, between 2.5×10¹¹ to 3×10¹¹, between 3×10¹¹ to 4×10¹¹, between 4×10¹¹ to 5×10¹¹, between 5×10¹¹ to 6×10¹¹, between 6×10¹¹ to 7×10¹¹, between 7×10¹¹ to 8×10¹¹, between 8×10¹¹ to 9×10¹¹, between 9×10¹¹ to 10×10¹¹, between 1×10¹² to 2×10¹², between 2×10¹² to 3×10¹², between 2.5×10¹² to 3×10¹², between 3×10¹² to 4×10¹², between 4×10¹² to 5×10¹², between 5×10¹² to 6×10¹², between 6×10¹² to 7×10¹², between 7×10¹² to 8×10¹², between 8×10¹² to 9×10¹², between 9×10¹² to 10×10¹², between 1×10¹³ to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 9×10¹³ to 10×10¹³ vector genomes. In some embodiments, a unit dose of 7m8-aflibercept of this disclosure is between 2.1×10¹¹ or between 2.1×10¹² vector genomes. In some embodiments, the unit dose of rAAV of this disclosure is between 10¹⁰ to 10¹³, between 10¹⁰ to 10¹¹, between 10¹¹ to 10¹², between 10¹² to 10¹³, or between 10¹³ to 10¹⁴ vector genomes.

In some embodiments, a unit dose of 7m8-aflibercept of this disclosure is between 1×10¹⁰ to 2×10¹⁰, between 2×10¹⁰ to 4×10¹⁰, between 3×10¹⁰ to 5×10¹⁰, between 4×10¹⁰ to 6×10¹⁰, between 5×10¹⁰ to 7×10¹⁰, between 6×10¹⁰ to 8×10¹⁰, between 7×10¹⁰ to 9×10¹⁰, between 8×10¹⁰ to 10¹¹, between 1×10¹¹ to 2×10¹¹, between 2×10¹¹ to 4×10¹¹, between 3×10¹¹ to 5×10¹¹, between 4×10¹¹ to 6×10¹¹, between 5×10¹¹ to 7×10¹¹, between 6×10¹¹ to 8×10¹¹, between 7×10¹¹ to 9×10¹¹, between 8×10¹¹ to 10×10¹¹, between 1×10¹² to 3×10¹², between 2×10¹² to 4×10¹², between 3×10¹² to 5×10¹², between 4×10¹² to 6×10¹², between 5×10¹² to 7×10¹², between 6×10¹² to 8×10¹², between 7×10¹² to 9×10¹², between 8×10¹² to 10×10¹², between 1×10¹³ to 5×10¹³, between 5×10¹³ to 10×10¹³, between 10¹² to 5×10¹², between 5×10¹² to 1×10¹³, between 7×10¹² to 1×10¹³, between 8×10¹² to 2×10¹³, between 9×10¹² to 2×10¹³, between 9×10¹² to 2×10¹³, between 9×10¹² to 4×10¹³, between 1×10¹³ to 3×10¹³, between 1×10¹³ to 2×10¹³, between 2×10¹³ to 3×10¹³, between 3×10¹³ to 4×10¹³, between 4×10¹³ to 5×10¹³, between 5×10¹³ to 6×10¹³, between 6×10¹³ to 7×10¹³, between 7×10¹³ to 8×10¹³, between 8×10¹³ to 9×10¹³, or between 8×10¹³ to 1×10¹⁴ vector genomes.

In some embodiments, the therapeutically effective amount of pharmaceutical compositions disclosed herein comprises between 2E12 to 6E12 vector genomes. In some embodiments, a unit dose comprises about 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, or 9.5E12 vector genomes. In some embodiments, a unit dose comprises between 1E12 to 1.5E12, between 1.5E12 to 2E12, between 2E12 to 2.5E12, between 2.5E12 to 3.0E12, between 3.0E12 to 3.5E12, between 3.5E12 to 4.0E12, between 4.0E12 to 4.5E12, between 4.5E12 to 5.0E12, between 5.0E12 to 5.5E12, between 5.5E12 to 6.0E12, between 6.0E12 to 6.5E12, between 6.5E12 to 7.0E12, between 7.0E12 to 7.5E12, between 7.5E12 to 8.0E12, between 8.0E12 to 8.5E12, between 8.5E12 to 9.0E12, between 9.0E12 to 9.5E12, or between 9.5E12 to 10E12 vector genomes. In some embodiments, a unit dose comprises at least 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, or 9.5E12 vector genomes. In some embodiments, a unit dose comprises no more than 1E12, 1.5E12, 2E12, 2.5E12, 3E12, 3.5E12, 4E12, 4.5E12, 5E12, 5.5E12, 6E12, 6.5E12, 7E12, 7.5E12, 8E12, 8.5E12, 9E12, 9.5E12, or 10E12 vector genomes.

In some embodiments, the total amount of 7m8-aflibercept injected into a human patient or subject within a period of 2 to 5 years or 5 to 10 years is not more than 10¹⁰ to 10¹³, 10¹⁰ to 10¹¹, 10¹¹ to 10¹², 10¹² to 10¹³, or 10¹³ to 10¹⁴ vector genomes, or no more than 1×10¹⁰ to 2×10¹⁰, 2x10¹⁰ to 4×10¹⁰, 3×10¹⁰ to 5×10¹⁰, 4×10¹⁰ to 6x10¹⁰, 5×10¹⁰ to 7×10¹⁰, 6×10¹⁰ to 8×10¹⁰, 7×10¹⁰ to 9×10¹⁰, 8×10¹⁰ to 10¹¹ , 1×10¹¹ to 2×10¹¹, 2×10¹¹ to 4×10¹¹, 3×10¹¹ to 5×10¹¹, 4×10¹¹ to 6×10¹¹, 5×10¹¹ to 7×10¹¹, 6×10¹¹ to 8×10¹¹, 7×10¹¹ to 9×10¹¹, 8×10¹¹ to 10×10¹¹, 1×10¹² to 3×10¹², 2×10¹² to 4×10¹², 3×10¹² to 5×10¹², 4×10¹² to 6×10¹², 5×10¹² to 7×10¹², 6×10¹² to 8×10¹², 7×10¹² to 9×10¹² , 8×10¹² to 10×10¹² , 1×10¹³ to 5×10¹³, 5×10¹³ to 10×10¹³ , 10¹² to 5×10¹², 5×10¹² to 1×10¹³, 7×10¹² to 1×10¹³ , 8×10¹² to 2×10¹³, 9×10¹² to 2×10¹³, 9×10¹² to 2×10¹³, 9×10¹² to 4×10¹³, 1×10¹³ to 3×10¹³, 1×10¹³ to 2×10¹³, 2×10¹³ to 3×10¹³, 3×10¹³ to 4×10¹³, 4×10¹³ to 5×10¹³, 5×10¹³ to 6×10¹³ 6×10¹³ to 7×10¹³, 7×10¹³ to 8×10¹³, 8×10¹³ to 9×10¹³, or 8×10¹³ to 1×10¹⁴ vector genomes.

In some embodiments, rAA V.7m8-aflibercept virion or a pharmaceutical composition thereof can be administered as a single dose or a one-time dose. In some embodiments, more than one administration may be employed to achieve the desired level of gene expression over a sustained period of various intervals, e.g., not more than once in at least 2 years, or at least 3, 4, 5, 6, 7, 8, 9, 10, or more years. In some embodiments, intravitreal injection of 7m8-aflibercept obviates a patient's need to receive an aflibercept protein injection for at least 1 year, 1.5. years, or at least 2, 3, 4, 5, 6, 7, 8, 9, 10, 20, 30 or more years.

### EXAMPLES

### Example 1: Efficacy evaluation of rAAV2.7m8-aflibercept in monkeys

It has been postulated that delivery of a therapeutic transgene (or payload) into target cells or tissue via gene therapy is largely dependent on AAV capsid proteins and their role in targeting the AAV virus to relevant or target cells in a primate or human subject. It has also been reported that the 7m8 variant shows increased infectivity or targeting of retinal cells when injected intravitreally. Thus, one would expect the 7m8 variant to work similarly in targeting various therapeutic transgenes to retinal cells when injected intravitreally.

Objective: To assess the efficacy of a rAAV2.7m8 comprising a nucleic acid sequence that encodes aflibercept with that of a rAAV2.7m8 comprising a nucleic acid sequence that encodes sVEGFR-1 following intravitreal (IVT) administration of each gene therapy at a dose of 2 × 10¹² vg to inhibit the development of choroidal neovascularization (CNV) induced by laser photocoagulation in African green monkeys. The nucleic acid sequence of sVEGFR-1 (also referred to as sFLT-1) is publicly available, e.g., as described in U.S. Patent Pub. 2014/0371438.

**FIG. 5** illustrates the nucleic acid sequence of aflibercept. **FIG. 6** illustrates the nucleic acid sequence of sFLT-1 (SEQ ID NO: 3).

CNV lesion model in monkeys is a generally accepted as and a widely used standard primate model for assessing potential efficacy of therapies for treating eye diseases associated with neovascularization, such as wet AMD.

Monkeys underwent baseline screening to assess ocular and general health by tonometry, slit lamp biomicroscopy, fundoscopy, color fundus photography (CFP), fluorescence angiography (FA) and optical coherence tomography (OCT). Monkeys with normal findings were enrolled in the study and randomized into three treatment groups by baseline body weight and gender **(Table 1).** Atropine 1% ophthalmic ointment was applied following baseline exam.

As used herein, rAAV2.7m8-sVEGFR-1 comprises rAAV2 comprising the 7m8 insertion between positions 587 and 588 in capsid protein VP1 of rAAV2 and a nucleic acid sequence encoding sVEGFR-1.

**Table 1: Treatment Assignment**

| **Group** | **N** | **Treatment OU** | **Route** | **Dose (µL)** | **Laser OU** | **Slit lamp & CFP** | **FA** & OCT** | **Terminus & tissue collection** |
|---|---|---|---|---|---|---|---|---|
| 1 | 6 | AAV2.7m8-aflibercept | IVT; Day 0 | 1x100 µL | Day 56 | | | |
| 2 | 6 | Vehicle | IVT; Day 0 | 1x100 µL | Day 56 | | | |
| 3 | 6 | AAV2.7m8-sVEGFR-1 | IVT; Day 0 | 1x100 µL | Day 56 | | | |
| 4 | 3 | Eylea^{®} (aflibercept) | IVT; Day 56 | 1x30 µL | Day 56 | Day 84 | Baseline, day 70 & 84 | |

| | | | | | | | | |
|---|---|---|---|---|---|---|---|---|
| * CFP was additionally performed on day 21 if day 14 images did not reveal clear images of stabilized blebs. Slit lamp was performed prior to laser on day 56 but not immediately post-injection on day 0. | | | | | | | | |

On study day 0, monkeys of groups 1-3 received intravitreal (IVT) AAV2.7m8-aflibercept, vehicle, or AAV2.7m8-sVEGFR-1 OU in accordance with the treatment schedule **(Table 1)**. Prior to IVT dosing, topical local anesthesia was administered (0.5% proparacaine) and eyes can be disinfected with 5% Betadine and rinsed with sterile normal saline. IVT injections were administered using a 31-gauge 0.5-inch needle placed 2 mm posterior to the limbus in the inferior temporal quadrant, targeting the central vitreous.

On study day 56, monkeys of group 4 received IVT aflibercept (EYLEA^{®}, 30 µL of 40 mg/mL/eye) OU immediately after laser treatment, following identical IVT injection procedures as those of groups 1-3 with the exception of the reduced volume of injection (30 µL). IVT administration of aflibercept is a clinical standard of care for CNV and therefore employed as a positive control in this study. The dose was adjusted to accommodate the relatively smaller volume of vitreous in the African green monkey (2.7 mL) in comparison with human (4 mL).

All IVT injections were followed by topical administration of 0.3% ciprofloxacin, or equivalent antibiotic ophthalmic solution, and 1% atropine sulfate ointment.

On Day 56 CNV was induced between temporal vascular arcades with laser bums. Nine laser spots were symmetrically placed in each eye by an ophthalmologist employing an Iridex Oculight TX 532 nm laser with a laser duration of 100 ms, spot size 50 µm, power 750 mW. Laser spots were applied using a 0.9x contact laser lens. The target location of laser spots was mapped by a trained ophthalmologist on color fundus images obtained prior to laser treatment (and subsequent to bleb placement) for reference during laser spot placement. Color fundus photography was performed immediately after the laser treatment to document the laser lesions. Any spots demonstrating severe retinal/subretinal hemorrhage immediately post-laser was excluded from analyses. **FIG. 1** illustrates an exemplary fundus photograph of an eye of a non-human primate after induction of CNV lesions by laser irradiation without treatment.

Bilateral color fundus images of the retina were captured with 50 degree of view centered on the fovea using a Topcon TRC-50EX retinal camera with Canon 6D digital imaging hardware and New Vision Fundus Image Analysis System software. FA was performed with intravenous administration of 0.1 mL/kg of 10% sodium fluorescein. Fluorescein leakage in angiograms of CNV lesions was graded I-IV **(Table 2)** by a masked ophthalmologist assessing composites generated after uniform adjustment of image intensity. Lesion grading assessment was confirmed on images of fundus by two other trained ophthalmologists. Image fluorescence densitometry analysis of late-stage raw angiograms can also be performed using ImageJ software.

**Table 2: Laser lesion grading scales**

| Lesion Grade | Definition |
|---|---|
| I | No hyperfluorescence - Compare pre-FA with 30 sec post-FA. Look for absence of hyperfluorescence in lesion |
| II | Hyperfluorescence without leakage - Compare 30 sec FA with 3 and 6 min FA. |
| | Look for hyperfluoresence without significant residual staining in 6 min FA. |
| III | Hyperfluorescence early or mid-transit and late leakage - Compare 30 sec FA with 3 and 6 min FA. Look for significant residual staining in lesion at 6 min FA. |
| IV | Hyperfluorescence early or mid-transit and late leakage extending beyond the borders of the treated area - Compare 30 sec FA with 3 and 6 min FA. Look for consistent staining beyond the border of the lesion as seen in 30 sec FA. |

Subjects were assessed twice daily for general wellbeing. Detailed observations were performed once weekly. Body weights were obtained at the time of baseline screening and every two weeks during the in-life study.

All animals were euthanized with pentobarbital after confirming the quality of fundus imaging on Day 85, or shortly thereafter, pending review of images. Animals were euthanized with pentobarbital and globes enucleated. Excess orbital tissue was trimmed and both OD and OS globes was flash frozen in liquid nitrogen then dissected along frozen tissue planes at room temperature to isolate vitreous and retinal with choroidal sub-tissues. After collection of vitreous, 5 mm punches of neural retina with RPE/choroid were taken from the macula and superior, inferior, temporal and nasal regions. The retina with underlying RPE/choroidal tissues from each punch was transferred to pre-tared labeled cryotubes, and weighed and flash frozen in liquid nitrogen. Before and after collection of the punch biopsies, a photograph of the flat mounted retina was taken with indication of orientation to document the regions from which the punches were collected.

Statistical methods: A Fisher's exact test was used to evaluate incidence of different lesion grades. A two way ANOVA with repeated measures followed by Tukey-Kramer test or a contrast procedure was used to analyze the OCT CNV complex area and angiogram image densitometry data. Non-parametric tests were applied if the data was not normally distributed and has an unequal variance. P value of 0.05 or less was considered statistically significant.

CNV lesions were induced by laser irradiation immediately after injection in each group of test subjects (monkeys), and color fundus photography was used to grade each lesion on a scale of I-IV. **FIG. 2** illustrates an exemplary fundus photography used to evaluate the lesions of monkeys at day 70 following treatment with either AAV2.7m8-aflibercept or the vehicle control intravitreally at a dose of 2.1 × 10¹² vg. A monkey treated with AAV2.7m8-aflibercept showed lesions graded II and III and no grade IV lesions, while a monkey treated with the vehicle control showed more lesions graded IV. Similar data from multiple monkeys were pooled and plotted for quantitative analysis for each test group described further below **(****FIG. 3** and **4****).**

**FIG. 3** illustrates a plot of the percentage of grade IV lesions at day 14 and at day 28 of monkeys of group 4 injected intravitreally with either EYLEA^{®} as a positive control (or aflibercept fusion protein without the gene therapy approach) or a vehicle control comprising formulation buffer only. 30 µL of 40 mg/mL/eye EYLEA^{®} OU was administered immediately after laser irradiation, following identical IVT injection procedures as used for groups 1-3 **(Table 1)** with the exception of the reduced volume of injection (30 µL) to accommodate the relatively smaller volume of vitreous in African green monkey (2.7 mL) in comparison with human (4 mL). IVT administration of EYLEA^{®} is a clinical standard of care for CNV and therefore employed as a positive control in this study. Data from multiple monkeys were averaged and plotted for quantitative analysis. Animals treated with intravitreal injection of EYLEA^{®} showed a significant decrease in the amount of grade IV lesions as compared to administration of vehicle alone by intravitreal injection, as measured by the fundus images collected at day 14 and at day 28 post injection.

**FIG. 4** illustrates a plot of the percentage of grade IV lesions at day 14 and at day 28 of monkeys (groups 1-3 in **Table 1)** injected intravitreally with either rAAV2.7m8-aflibercept (which is a gene therapy that comprises rAAV2 with the 7m8 variant capsid protein and the aflibercept nucleic acid sequence); or AAV2.7m8-sVEGFR-1 (which is a gene therapy that comprises rAAV2 with the 7m8 variant capsid protein and the sVEGFR-1 nucleic acid sequence), each at a dose of 2.1 × 10¹² vg, or a vehicle control comprising formulation buffer only. CNV lesions were induced by laser irradiation, and color fundus photography was used to grade each lesion on a scale of I-IV. Measurements of percentage of grade IV lesions were then pooled and plotted. Monkeys treated with intravitreal injection of rAAV2.7m8-aflibercept showed a significant decrease in the amount of grade IV lesions as compared to the vehicle control alone for the fundus images collected at day 14 and at day 28. In contrast, and unexpectedly, monkeys treated with intravitreal injection of rAAV2.7m8-sVEGFR-1 showed little to no reduction of grade IV CNV lesions as compared to the vehicle control. This data suggest that capsid variation and/or route of administration may not work for all transgenes, which is contrary to what many had thought previously, and that the properties of the transgene can play a significant role in the efficacy of AAV gene therapy. Such in vivo data in monkeys also illustrated that treatment of primates with rAAV2.7m8-aflibercept led to fewer grade IV lesions as compared to monkeys that received the vehicle control only, suggesting that rAAV2.7m8-aflibercept can be a viable gene therapy option for humans.

These in vivo studies of rAAV2.7m8-aflibercept showed that rAAV2 comprising the 7m8 variant capsid protein can be effective as a gene therapy for delivering a nucleic acid sequence that encodes aflibercept to retinal cells of a subject by intravitreal injection, and results in an expression of active aflibercept in vivo such that it exerts a therapeutic effect, i.e., reduction of CNV lesions, at levels that are similar to the therapeutic effect of the EYLEA^{®} positive control.

Certain embodiments of the invention are set out in the following numbered paragraphs:
1. A method of treating an eye condition or disease, the method comprising administering a unit dose of a pharmaceutical composition by intravitreal injection to an eye of a primate subject in need thereof, wherein the pharmaceutical composition comprises:
   (a) a rAAV2 variant comprising an amino acid sequence LGETTRP inserted between positions 587 and 588 of capsid protein VP1, and a nucleic acid sequence encoding a polypeptide having at least 80% homology to aflibercept, a functional fragment, or variant thereof, and
   (b) a pharmaceutically acceptable excipient.
2. The method of paragraph 1, wherein the eye condition or disease is neovascular (wet) age-related macular degeneration (AMD), macular edema following retinal vein occlusion, diabetic macular edema (DME), or diabetic retinopathy associated with DME.
3. The method of paragraph 1, wherein the eye condition or disease is choroidal neovascularization or wet AMD.
4. The method of any of paragraphs 1-3, wherein the unit dose comprises between 1E12 to 1E13 vector genomes.
5. The method of any of paragraphs 1-3, wherein the unit dose comprises between 2E12 to 6E12 vector genomes.
6. The method of paragraph 1, wherein the unit dose comprises a volume that is not more than 100 µL.
7. The method of paragraph 1, wherein the unit dose comprises a volume that is not more than 50 µL.
8. The method of paragraph 1, wherein the subject is a non-human primate.
9. The method of paragraph 1, wherein the subject is a human.
10. The method of paragraph 1, wherein the subject is responsive to aflibercept.
11. The method of paragraph 1, wherein the subject has been pre-treated with aflibercept.
12. The method of paragraph 1, wherein the administering by intravitreal injection occurs not more than once in at least 2 years.
13. The method of paragraph 1, wherein the administering by intravitreal injection occurs not more than once in at least 5 years.
14. The method of paragraph 1, wherein the administering by intravitreal injection is a one-time administration.
15. The method of paragraph 1, wherein the pharmaceutical composition is a suspension.
16. The method of paragraph 15, further comprising agitating the suspension to ensure even distribution of the suspension prior to the administering step.
17. The method of paragraph 1, further comprising warming the pharmaceutical composition to room temperature prior to the administering step.
18. A pharmaceutical composition comprising a suspension, comprising: a rAAV2 variant comprising an amino acid sequence LGETTRP inserted between positions 587 and 588 of a capsid protein VP1, and a nucleic acid sequence encoding a polypeptide having at least 80% homology to aflibercept, a functional fragment, or variant thereof.
19. The pharmaceutical composition of paragraph 18, wherein the nucleic acid sequence comprises a sequence of SEQ ID NO: 2.
20. The pharmaceutical composition of paragraph 18, wherein a unit dose of the composition comprises between 1E12 to 1E13 vector genomes.
21. The pharmaceutical composition of paragraph 18, wherein a unit dose of the pharmaceutical composition comprises 2E12 to 6E12 vector genomes.
22. A kit, comprising the pharmaceutical composition of paragraph 18 and a solution for diluting the pharmaceutical composition.
23. The kit of paragraph 22, wherein the pharmaceutical composition comprises not more than 1 mL.
24. The kit of paragraph 22, wherein the pharmaceutical composition comprises not more than 0.5-1.0 mL.
25. The kit of paragraph 22, wherein the pharmaceutical composition comprises less than or equal to 0.5 mL.
26. The kit of paragraph 22, wherein the solution comprises a buffer, salt, alcohol, a surfactant, or any combination thereof.
27. The kit of paragraph 22, further comprising a syringe.
28. A method of treating an eye condition or disease, the method comprising:
   (a) agitating a refrigerated suspension, the suspension comprising: a rAAV2 variant comprising an amino acid sequence LGETTRP inserted between positions 587 and 588 of a capsid protein VP1, and a nucleic acid sequence encoding a polypeptide having at least 80% homology to aflibercept, a functional fragment, or variant thereof; and
   (b) administering a volume of the refrigerated suspension to an eye of a human subject via intravitreal injection.
29. The method of paragraph 28, wherein the subject is characterized as having been pre-treated with aflibercept.
30. The method of paragraph 28, wherein the subject is responsive to aflibercept.
31. The method of paragraph 28, wherein the refrigerated suspension comprises no more than 1 mL of the solution.
32. The method of paragraph 28, wherein the refrigerated suspension comprises no more than 0.5 mL.
33. The method of paragraph 28, wherein the volume administered to the subject is not more than 50 µL.
34. The method of paragraph 28, wherein the volume administered to the subject is not more than 100 µL.
35. The method of paragraph 28, wherein the volume comprises a unit dose of between 1E12 to 1E13 vector genomes.
36. The method of paragraph 28, wherein the volume comprises a unit dose of between 2E12 to 6E12 vector genomes.
37. The method of paragraph 28, wherein the administering step occurs not more than once in at least 2 years.
38. The method of paragraph 28, wherein the administering step is a one-time injection.
39. The method of paragraph 28, further comprising assaying the subject for responsiveness to aflibercept before administering the composition.
40. The method of paragraph 28, wherein the suspension comprises a pharmaceutically acceptable excipient.
41. The method of paragraph 40, wherein the excipient comprises a surfactant or a stabilizer.
42. The method of paragraph 41, wherein the surfactant is selected from polysorbates, sodium dodecyl sulfate, sodium lauryl sulfate, lauryl dimethyl amine oxide, polyethoxylated alcohols, polyoxyethylene sorbitan, octoxynol, Brij, pluronic, and polyoxyl castor oil.
43. The method of paragraph 40, wherein the pharmaceutically acceptable excipient comprises phenol, mannitol, sorbitol, or sodium chloride.
44. The method of paragraph 28, wherein the eye condition or disease is neovascular (wet) age-related macular degeneration (AMD), macular edema following retinal vein occlusion, diabetic macular edema (DME), or diabetic retinopathy associated with DME.
45. The method of paragraph 28, wherein the eye condition or disease is choroidal neovascularization or wet AMD.
46. The method of paragraph 28, further comprising warming the suspension to room temperature before administration.

## Claims

1. A pharmaceutical composition comprising:
(a) an rAAV having a variant capsid protein comprising an amino acid sequence insertion of 5 to 11 amino acids in the solvent-accessible portion of the VP1 capsid protein GH loop, which insertion confers increased infectivity of retinal cells, and a nucleic acid sequence encoding a polypeptide having at least 95% homology to aflibercept, and
(b) a pharmaceutically acceptable excipient;
for use in a method of treating an eye condition or disease, the method comprising administering a unit dose of the pharmaceutical composition by intravitreal injection to an eye of a primate subject in need thereof.

2. The pharmaceutical composition for use according to claim 1, wherein the rAAV is selected from AAV1, AAV2, AAV2.5, AAV3, AAV4, AAV5, AAV6, AAV7, AAV8, AAV9, AAV10, AAV11, AAV12, rh10, AAV-DJ, and any hybrid or chimeric AAV thereof.

3. The pharmaceutical composition for use according to any one of the preceding claims, wherein the amino acid sequence insertion is selected from LGETTRP (7m8), NETITRP, KAGQANN, KDPKTTN, KDTDTTR, RAGGSVG, AVDTTKF, and STGKVPN.

4. The pharmaceutical composition for use according to claim 1, wherein the insertion comprises an amino acid sequence LGETTRP (7m8):
(i) inserted in a rAAV2 between positions 587 and 588 of the AAV2 capsid protein VP1;
(ii) inserted in a rAAV1 between amino acids 590 and 591 of the AAV1 capsid protein VP1;
(iii) inserted in a rAAV5 between amino acids 575 and 576 of the AAV5 capsid protein VP1;
(iv) inserted in a rAAV6 between amino acids 590 and 591 of the AAV6 capsid protein VP1;
(v) inserted in a rAAV7 between amino acids 589 and 590 of the AAV7 capsid protein VP1;
(vi) inserted in a rAAV8 between amino acids 590 and 591 of the AAV8 capsid protein VP1;
(vii) inserted in a rAAV9 between amino acids 588 and 589 of the AAV9 capsid protein VP1; or
(viii) inserted in a rAAV10 between amino acids 589 and 590 of the AAV10 capsid protein VP1.

5. The pharmaceutical composition for use according to any one of the preceding claims, wherein the eye condition or disease is neovascular (wet) age-related macular degeneration (AMD), macular edema following retinal vein occlusion, diabetic macular edema (DME), or diabetic retinopathy associated with DME.

6. The pharmaceutical composition for use according to any one of claims 1-4, wherein the eye condition or disease is choroidal neovascularization or wet AMD.

7. The pharmaceutical composition for use according to any one of the preceding claims, wherein the polypeptide encoded by the nucleic acid comprises a sequence having 100% homology to aflibercept.

8. The pharmaceutical composition for use according to any one of the preceding claims, wherein the unit dose is: (a) between 1E12 to 1E13 vector genomes; (b) between 2E12 to 6E12 vector genomes; (c) between 1E9 to 3E13 vector genomes; (d) between 1E10 to 3E12 vector genomes; or (e) between 1E8 to 3E14 vector genomes.

9. The pharmaceutical composition for use according to any one of the preceding claims, wherein the unit dose is: (a) in a volume that is not more than 100 µL; or (b) in a volume that is not more than 50 µL.

10. The pharmaceutical composition for use according to any one of the preceding claims, wherein the subject is a human.

11. The pharmaceutical composition for use according to any one of the preceding claims, wherein the subject is responsive to aflibercept.

12. The pharmaceutical composition for use according to any one of the preceding claims, wherein the subject has been pre-treated with aflibercept.

13. The pharmaceutical composition for use according to any one of the preceding claims, wherein the administering by intravitreal injection occurs not more than once in at least 2 years, optionally not more than once in at least 5 years.

14. The pharmaceutical composition for use according to any one of claims 1-12, wherein the administering by intravitreal injection is a one-time administration.

15. The pharmaceutical composition for use according to any one of the preceding claims, wherein: (a) the pharmaceutical composition is a suspension, optionally further comprising agitating the suspension to ensure even distribution of the suspension prior to the administering step; and/or (b) the method further comprises warming the pharmaceutical composition to room temperature prior to the administering step.
